# EUROPEAN PATENT APPLICATION

(11) **EP 4 501 917 A1**
(43) Date of publication of application: **05.02.2025**
(21) Application number: 23781235.9
(22) Date of filing: 22.03.2023
(51) Int. Cl.: C07D 241/44, A61K 31/498, A61P 35/00, C07D 413/12, C07D 417/12, C07D 403/12, A23L 33/10

(54) **3,4-DIHYDROQUINOXALINE-2-CARBOXIMIDE DERIVATIVE COMPOUND AND PHARMACEUTICAL COMPOSITION COMPRISING SAME FOR PREVENTING OR TREATING CANCER DISEASE**

(30) Priority: 31.03.2022 KR 20220040563
(71) Applicant: Daegu-Gyeongbuk Medical Innovation Foundation, Daegu 41061 (KR)
(72) Inventor: CHIN, Jungwook, Daegu 41061 (KR); JUNG, Kyungjin, Daegu 41061 (KR); KIM, Jina, Daegu 41061 (KR); KIM, Suhui, Daegu 41061 (KR); KIM, Seokkyu, Daegu 41061 (KR); HWANG, Jun Yeon, Daegu 41061 (KR); YU, Ji Hoon, Daegu 41061 (KR); LEE, Heejin, Daegu 41061 (KR); JEON, Yong Hyun, Daegu 41061 (KR); CHO, Sung Jin, Daegu 41061 (KR); KADAYAT, Tara Man, Daegu 41061 (KR); LEE, Sang Bong, Daegu 41061 (KR)
(74) Representative: Mewburn Ellis LLP
(86) International application number: PCT/KR2023/003810
(87) International publication number: WO 2023/191379

(57) **Abstract**

The present disclosure relates to a 3,4-dihydroquinoxaline-2-carboximide derivative compound and a pharmaceutical composition comprising same for preventing or treating a cancer disease. The compound has an excellent effect of selectively inhibiting histone deacetylase (HDAC) 8 among HDACs, and thus can be useful as a selective inhibitor of HDAC8 or a pharmaceutical composition for preventing or treating a cancer disease.

## Description

### Technical Field

The present disclosure relates to a 3,4-dihydroquinoxaline-2-carboxamide derivative compound and a pharmaceutical composition for preventing or treating cancer disease including the same.

### Background Art

Histone deacetylase (hereinafter referred to as HDAC) is an enzyme that removes the acetyl group from an amino group of the N-terminal lysine tail of histones, which is known to have various effects on non-histone proteins, and is involved in important cellular activities such as cell growth cycle regulation, differentiation, and cancer formation. In particular, it has recently been revealed that it is overexpressed under harsh environmental conditions such as hypoxia, low glucose, and cell tumorigenesis and plays a role in promoting cell proliferation by inhibiting the expression of cell proliferation inhibitory factors, and is recognized as an important regulatory factor in the control of tumorigenicity and differentiation of cells. HDAC can be broadly classified into four groups depending on its function and DNA sequence similarity, and as of now, it consists of 18 groups. Among them, HDAC 8 belongs to class I, and within the class, HDACs 1, 2, and 3 are mainly found in the nucleus, while HDAC8 exists in both the nucleus and cytoplasm. HDAC 8 is overexpressed in several cancers and has been reported to be associated with various diseases, including inflammatory diseases, and is emerging as an important target for the treatment of various diseases.

HDAC inhibitors currently approved by the FDA include Novartis' multiple myeloma treatment 'Farydak (panobinostat)', BMS' cutaneous T-cell lymphoma treatment 'Istodax (romidepsin)', Merck's cutaneous T-cell lymphoma treatment `Zolinza (vorinostat)', Acrotech's peripheral T-cell lymphoma treatment 'Beleodaq (belinostat)', and etc. Most of the HDAC inhibitors developed so far have been approved for the treatment of specific cancers, but their effectiveness is limited and may cause unwanted side effects, so more improved HDAC selective inhibitors are needed.

### Disclosure of the Invention

### Technical Goals

One embodiment is intended to provide a novel 3,4-dihydroquinoxaline-2-carboxamide derivative compound.

Another embodiment is intended to provide a method of preparing a 3,4-dihydroquinoxaline-2-carboxamide derivative compound.

Another embodiment is intended to provide a pharmaceutical composition for preventing or treating cancer disease including a 3,4-dihydroquinoxaline-2-carboxamide derivative compound.

Another embodiment is intended to provide a health functional food composition for preventing or ameliorating cancer disease, including a 3,4-dihydroquinoxaline-2-carboxamide derivative compound.

Another embodiment is intended to provide a method of treating cancer disease, including administering the compound represented by Chemical Formula 1, a steroisomer thereof, a hydrate thereof, or a pharmaceutically acceptable salt thereof to an individual or subject in need thereof.

Another embodiment is intended to provide a 3,4-dihydroquinoxaline-2-carboxamide derivative compound, a stereoisomer thereof, a hydrate thereof, or a pharmaceutically acceptable salt thereof for use in the treatment of cancer disease.

Another embodiment is intended to provide a use of a 3,4-dihydroquinoxaline-2-carboxamide derivative compound, a stereoisomer thereof, a hydrate thereof, or a pharmaceutically acceptable salt thereof, in the manufacture of a drug for treating cancer disease.

### Technical Solutions

One embodiment provides a compound represented by the following Chemical Formula 1, a stereoisomer thereof, a hydrate thereof, or a salt thereof.

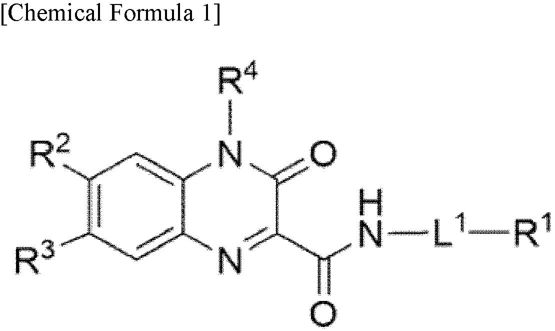

In the Chemical Formula 1,
R¹ is unsubstituted or substituted Cs-scycloalkyl, unsubstituted or substituted C₆₋₁₀aryl, unsubstituted or substituted heteroaryl of 5 to 8 atoms containing one or more heteroatoms selected from the group consisting of N, O and S, unsubstituted or substituted fused heteroaryl of 8 to 10 atoms containing one or more heteroatoms selected from the group consisting of N, O and S, or C₁₋₁₅alkenyl,
wherein the substituted Cs-scycloalkyl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₁₋₈alkoxycarbonyl, C₁₋₈alkoxycarbonylC₁₋₅alkyl, and C₁₋₈alkylcarbonyloxy,
the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₆₋₁₀aryl, C₁₋₈alkoxycarbonyl, C₁₋₈alkoxycarbonylC₁₋₅alkyl, C₁₋₈alkylcarbonyloxy, and heteroaryl of 5 to 8 atoms containing one or more heteroatoms selected from the group consisting of N, O and S,
the substituted heteroaryl of 5 to 8 atoms is substituted with halogen, or C₆₋₁₀aryl substituted with halogen, and
the substituted fused heteroaryl of 8 to 10 atoms is substituted with one or more substituents selected from the group consisting of -OH, halogen, C₁₋₈alkoxycarbonyl, C₁₋₈alkoxycarbonylC₁₋₅alkyl, and C₁₋₈alkylcarbonyloxy;
R² and R³ are each independently -H, halogen, or C₁₋₁₀alkyl;
R⁴ is C₁₋₈alkyl, or C₃₋₁₀alkyl substituted with two or more -OH; and
L¹ may be a bond, or C₁₋₁₀alkylene.

Another embodiment provides a method of preparing a compound represented by Chemical Formula 1, including, as shown in the following Reaction Scheme 1,
reacting a compound represented by Chemical Formula 1A with a hydroxide ion (OH⁻) to prepare a compound represented by Chemical Formula 1B; and
reacting a compound represented by Chemical Formula 1B with a compound represented by Chemical Formula 1C to prepare a compound represented by Chemical Formula 1.

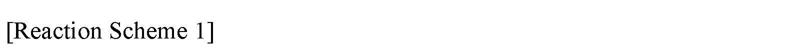

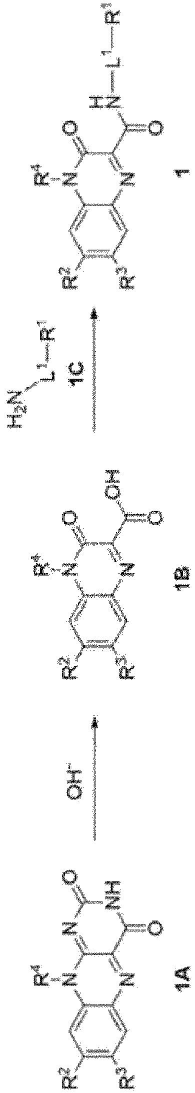

Another embodiment provides a pharmaceutical composition for preventing or treating cancer disease including the compound represented by Chemical Formula 1, a steroisomer thereof, a hydrate thereof, or a pharmaceutically acceptable salt thereof, as an active ingredient.

Another embodiment provides a health functional food composition including the compound represented by Chemical Formula 1, a steroisomer thereof, a hydrate thereof, or a pharmaceutically acceptable salt thereof, as an active ingredient.

Another embodiment provides a method of treating cancer disease, including administering the compound represented by Chemical Formula 1, a steroisomer thereof, a hydrate thereof, or a pharmaceutically acceptable salt thereof to an individual or subject in need thereof.

Another embodiment provides the compound represented by Chemical Formula 1, a steroisomer thereof, a hydrate thereof, or a pharmaceutically acceptable salt thereof for use in the treatment of cancer disease.

Another embodiment provides a use of the compound, a steroisomer thereof, a hydrate thereof, or a pharmaceutically acceptable salt thereof, in the manufacture of a drug for treating cancer disease.

### Advantageous Effects

The present disclosure relates to a 3,4-dihydroquinoxaline-2-carboxamide derivative compound and a pharmaceutical composition including the same for preventing or treating cancer disease, and since the compound has an excellent effect of selectively inhibiting HDAC8 among histone deacetylases (HDACs), it may be usefully used as a selective inhibitor of HDAC8 or a pharmaceutical composition for preventing or treating cancer disease.

### Brief Description of Drawings

FIG. 1 is a diagram showing results of confirming a growth inhibitory effect on prostate cancer cells PC-3 using PCI-34051, known as an HDAC8 inhibitor, and N-(2-(5-hydroxy-1H-indol-3-yl)ethyl)-6,7-dimethyl-3-oxo-4-((25,3 S,4R)-2,3,4,5-tetrahydroxypentyl)-3,4-dihydroquinoxaline-2-carboxamide (compound of Example 27) and N-(4-fluoro-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide (compound of Example 35).
FIG. 2 is a diagram showing results of confirming anticancer efficacy by measuring a volume of a tumor extracted from a mouse model transplanted with a prostate cancer cell line PC-3 during an administration period of a compound of Example 27.
FIG. 3 is a diagram showing results of confirming anticancer efficacy by measuring a weight of a tumor extracted from a mouse model 12 days after administration of a compound of Example 27.
FIG. 4 is a diagram showing results of observing a decrease in tumor volume by photographing a tumor extracted from a mouse model 12 days after administration of a compound of Example 27.
FIG. 5 is a diagram showing results of measuring a change in body weight of a mouse model during an administration period of a compound of Example 27.
FIG. 6 is a diagram showing results of confirming a growth inhibitory effect on liver cancer cells Huh-7 using compounds of Example 27 and Example 35.
FIG. 7 is a diagram showing data evaluating a liver cancer effectiveness of a compound of Example 27.

### Best Mode for Carrying Out the Invention

Embodiments described herein may be modified into various other forms, and the technology according to one implementation is not limited to the embodiments described below. In addition, an embodiment of one implementation is provided to more completely explain the present disclosure to those with average knowledge in the art. Furthermore, throughout the specification, "comprising" or "including" a certain element means that other elements may be further included rather than excluding other elements, unless specifically stated to the contrary.

As used herein, numerical ranges include lower and upper limits and all values within that range, increments that are logically derived from the shape and width of the range being defined, all double restricted values, and all possible combinations of upper and lower limits of numerical ranges defined in different forms. As an example, if the content of the composition is limited to 10% to 80% or 20% to 50%, the numerical range of 10% to 50% or 50% to 80% should also be interpreted as described herein. Unless otherwise specified herein, values outside the numerical range that may occur due to experimental error or rounding of values are also included in the defined numerical range.

Hereinafter, unless otherwise specified herein, "about" may be considered a value within 30%, 25%, 20%, 15%, 10% or 5% of the specified value.

One embodiment provides a compound represented by the following Chemical Formula 1, a stereoisomer thereof, a hydrate thereof, or a salt thereof.

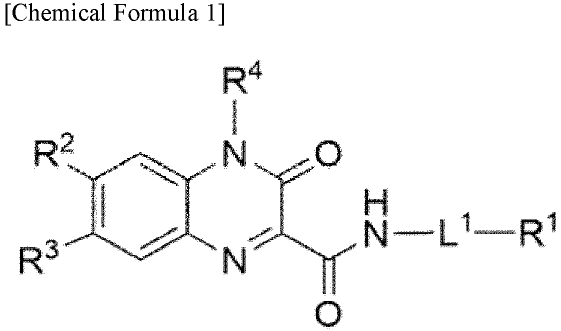

In the Chemical Formula 1,
R¹ is unsubstituted or substituted Cs-scycloalkyl, unsubstituted or substituted C₆₋₁₀aryl, unsubstituted or substituted heteroaryl of 5 to 8 atoms containing one or more heteroatoms selected from the group consisting of N, O and S, unsubstituted or substituted fused heteroaryl of 8 to 10 atoms containing one or more heteroatoms selected from the group consisting of N, O and S, or C₁₋₁₅alkenyl,
wherein the substituted Cs-scycloalkyl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₁₋₈alkoxycarbonyl, C₁₋₈alkoxycarbonylC₁₋₅alkyl, and C₁₋₈alkylcarbonyloxy,
the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₆₋₁₀aryl, C₁₋₈alkoxycarbonyl, C₁₋₈alkoxycarbonylC₁₋₅alkyl, C₁₋₈alkylcarbonyloxy, and heteroaryl of 5 to 8 atoms containing one or more heteroatoms selected from the group consisting of N, O and S,
the substituted heteroaryl of 5 to 8 atoms is substituted with halogen, or C₆₋₁₀aryl substituted with halogen, and
the substituted fused heteroaryl of 8 to 10 atoms is substituted with one or more substituents selected from the group consisting of -OH, halogen, C₁₋₈alkoxycarbonyl, C₁₋₈alkoxycarbonylC₁₋₅alkyl, and C₁₋₈alkylcarbonyloxy;
R² and R³ are each independently -H, halogen, or C₁₋₁₀alkyl;
R⁴ is C₁₋₈alkyl, or C₃₋₁₀alkyl substituted with two or more -OH; and
L¹ is a bond, or C₁₋₁₀alkylene.

In one embodiment, the alkyl, alkoxy, etc., may include straight or branched alkyl or alkoxy, etc.

In one embodiment, the halogen may be I, Br, Cl, or F.

In one embodiment, the term 'substituted' may mean that one or more, two or more, one or two substituents which are one or more, or two or more types among the listed substituents are substituted.

In one embodiment, the fused heteroaryl may include a ring in which aryl or cycloalkyl is fused to heteroaryl.

In one embodiment, the R¹ may be unsubstituted or substituted C₅₋₆cycloalkyl, unsubstituted or substituted C₆₋₈aryl, unsubstituted or substituted heteroaryl of 5 to 6 atoms containing one or more heteroatoms selected from the group consisting of N, O and S, unsubstituted or substituted fused heteroaryl of 8 to 10 atoms containing one or more heteroatoms selected from the group consisting of N, O and S, or C₅₋₁₅alkenyl,
wherein the substituted C₅₋₆cycloalkyl may be substituted with one or more substituents selected from the group consisting of halogen, -CONH-OH, -CH₂-CONH-OH, - CH₂CH₂-CONH-OH, C₁₋₈alkyl, C₁₋₈alkoxy, C₁₋₈haloalkyl, C₁₋₆alkoxycarbonyl, C₁₋₆alkoxycarbonylC₁₋₃alkyl, and C₁₋₆alkylcarbonyloxy,
the substituted C₆₋₈aryl may be substituted with one or more substituents selected from the group consisting of halogen, -CONH-OH, -CH₂-CONH-OH, -CH₂CH₂-CONH-OH, C₁₋₈alkyl, C₁₋₈alkoxy, C₁₋₈haloalkyl, C₆₋₈aryl, C₁₋₆alkoxycarbonyl, C₁₋₆alkoxycarbonylC₁₋₃alkyl, C₁₋₆alkylcarbonyloxy, and heteroaryl of 5 to 6 atoms containing one or more heteroatoms selected from the group consisting of N, O and S,
the substituted heteroaryl of 5 to 6 atoms may be substituted with halogen, or C₆₋₈aryl substituted with halogen, and
the substituted fused heteroaryl of 8 to 10 atoms may be substituted with one or more substituents selected from the group consisting of -OH, halogen, C₁₋₆alkoxycarbonyl, C₁₋₆alkoxycarbonylC₁₋₃alkyl, and C₁₋₆alkylcarbonyloxy.

Alternatively, in one embodiment, the R¹ may be unsubstituted or substituted C₅₋₆cycloalkyl, unsubstituted or substituted C₆₋₈aryl, unsubstituted or substituted heteroaryl of 5 to 6 atoms containing one or more heteroatoms selected from the group consisting of N, O and S, unsubstituted or substituted fused heteroaryl of 8 to 10 atoms containing one or more heteroatoms selected from the group consisting of N, O and S, or C₆₋₁₂alkenyl,
wherein the substituted C₅₋₆cycloalkyl may be substituted with one or more substituents selected from the group consisting of halogen, -CONH-OH, -CH₂-CONH-OH, - CH₂CH₂-CONH-OH, C₁₋₅alkyl, C₁₋₅haloalkyl, and C₁₋₅alkoxycarbonyl,
the substituted C₆₋₈aryl may be substituted with one or more substituents selected from the group consisting of halogen, -CONH-OH, -CH₂-CONH-OH, -CH₂CH₂-CONH-OH, C₁₋₅alkyl, C₁₋₅alkoxy, C₁₋₅haloalkyl, C₆aryl, C₁₋₅alkoxycarbonyl, C₁₋₅alkoxycarbonylC₁₋₃alkyl, C₁₋₅alkylcarbonyloxy, and heteroaryl of 5 to 6 atoms containing one or more heteroatoms selected from the group consisting of N, O and S,
the substituted heteroaryl of 5 to 6 atoms may be substituted with halogen, or C₆aryl substituted with halogen, and
the substituted fused heteroaryl of 8 to 10 atoms may be substituted with one or more substituents selected from the group consisting of -OH, halogen, C₁₋₅alkoxycarbonyl, C₁₋₅alkoxycarbonylC₁₋₃alkyl, and C₁₋₅alkylcarbonyloxy.

Alternatively, in one embodiment, the substituted Cs-scycloalkyl or C₅₋₆cycloalkyl may be substituted with halogen, -CONH-OH, -CH₂-CONH-OH, -CH₂CH₂-CONH-OH, C₁₋₅alkyl, C₁₋₄alkyl, C₁₋₃alkyl, C₁₋₂alkyl, -CH₃, C₁₋₅haloalkyl, C₁₋₄haloalkyl, C₁₋₃haloalkyl, C₁₋₂haloalkyl, C₁haloalkyl, C₁₋₅alkoxy, C₁₋₅alkoxy, C₁₋₄alkoxy, C₁₋₃alkoxy, C₁₋₂alkoxy, -OCH₃, C₁₋₅alkoxycarbonyl, C₁₋₄alkoxycarbonyl, C₁₋₃alkoxycarbonyl, C₁₋₂alkoxycarbonyl, - C(O)OCH₃, C₁₋₅alkoxycarbonylC₁₋₃alkyl, C₁₋₄alkoxycarbonylC₁₋₂alkyl, C₁₋₃alkoxycarbonylC₁₋₂alkyl, C₁₋₂alkoxycarbonylC₁₋₂alkyl, -CH₂CH₂C(O)OCH₃, -CH₂C(O)OCH₃, C₁₋₅alkylcarbonyloxy, C₁₋₄alkylcarbonyloxy, C₁₋₃alkylcarbonyloxy, C₁₋₂alkylcarbonyloxy, or - OC(O)CH₃, and may be substituted with one or more, or two or more, or one or two substituents, which are one or two or more types from these.

Alternatively, in one embodiment, the C₆₋₁₀aryl or C₆₋₈aryl may be substituted with halogen, -CONH-OH, -CH₂-CONH-OH, -CH₂CH₂-CONH-OH, C₁₋₅alkyl, C₁₋₄alkyl, C₁₋₃alkyl, C₁₋₂alkyl, -CH₃, C₁₋₅alkoxy, C₁₋₅alkoxy, C₁₋₄alkoxy, C₁₋₃alkoxy, C₁₋₂alkoxy, -OCH₃, C₁₋₅haloalkyl, C₁₋₄haloalkyl, C₁₋₃haloalkyl, C₁₋₂haloalkyl, C₁haloalkyl, C₆aryl(phenyl), C₁₋₅alkoxycarbonyl, C₁₋₄alkoxycarbonyl, C₁₋₃alkoxycarbonyl, C₁₋₂alkoxycarbonyl, -C(O)OCH₃, C₁₋₅alkoxycarbonylC₁₋₃alkyl, C₁₋₄alkoxycarbonylC₁₋₂alkyl, C₁₋₃alkoxycarbonylC₁₋₂alkyl, C₁₋₂alkoxycarbonylC₁₋₂alkyl, -CH₂CH₂C(O)OCH₃, -CH₂C(O)OCH₃, C₁₋₈alkylcarbonyloxy, C₁₋₄alkylcarbonyloxy, C₁₋₃alkylcarbonyloxy, C₁₋₂alkylcarbonyloxy, -OC(O)CH₃, or heteroaryl of 5 to 6 atoms containing one or more heteroatoms selected from the group consisting of N, O and S, and may be substituted with one or more, or two or more, or one or two substituents, which are one or two or more types from these.

Alternatively, in one embodiment, the substituted fused heteroaryl of 8 to 10 atoms may be substituted with -OH, halogen, C₁₋₅alkoxycarbonyl, C₁₋₄alkoxycarbonyl, C₁₋₃alkoxycarbonyl, C₁₋₂alkoxycarbonyl, -C(O)OCH₃, C₁₋₅alkoxycarbonylC₁₋₃alkyl, C₁₋₄alkoxycarbonylC₁₋₂alkyl, C₁₋₃alkoxycarbonylC₁₋₂alkyl, C₁₋₂alkoxycarbonylC₁₋₂alkyl, - CH₂CH₂C(O)OCH₃, -CH₂C(O)OCH₃, C₁₋₈alkylcarbonyloxy, C₁₋₄alkylcarbonyloxy, C₁₋₃alkylcarbonyloxy, C₁₋₂alkylcarbonyloxy, or -OC(O)CH₃, and may be substituted with one or more, or two or more, or one or two substituents, which are one or two or more types from these.

In one embodiment, the R¹ may be

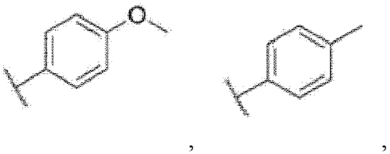

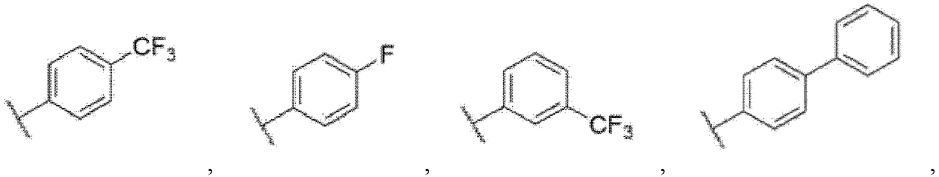

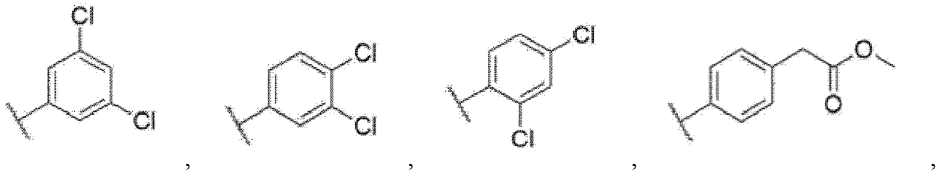

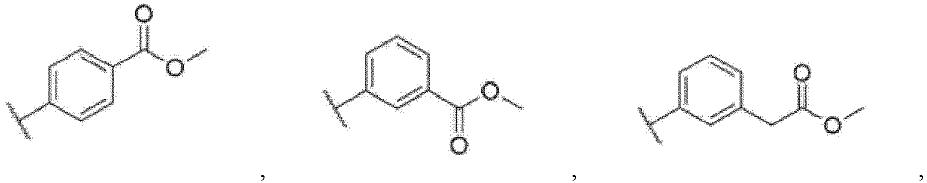

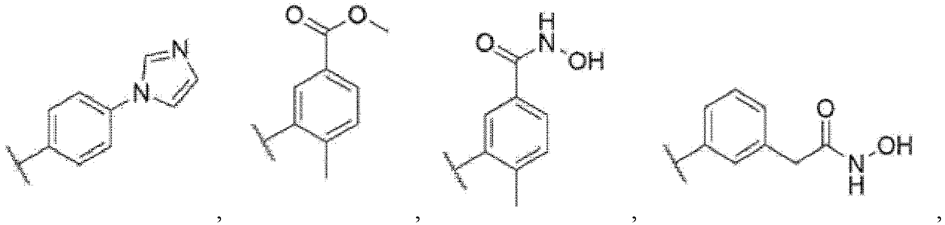

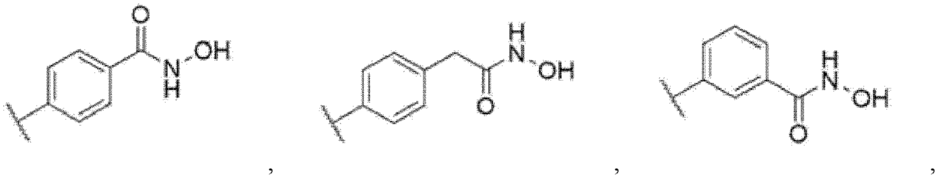

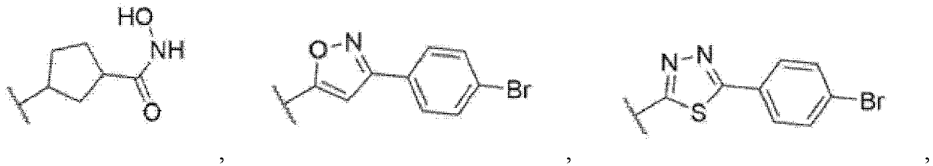

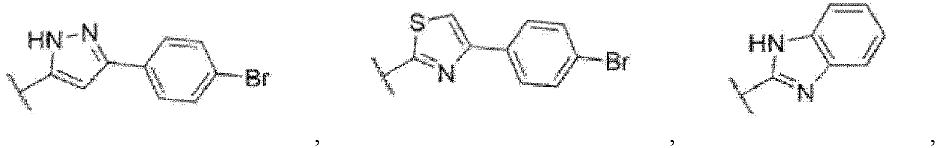

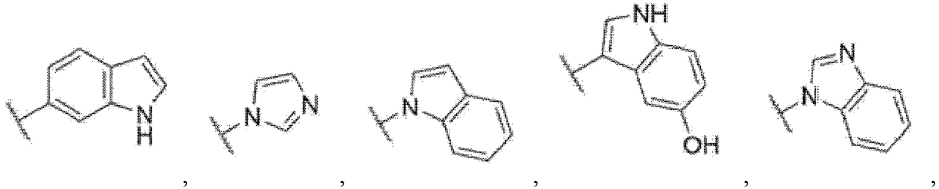

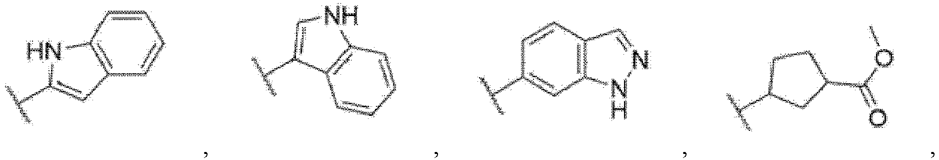

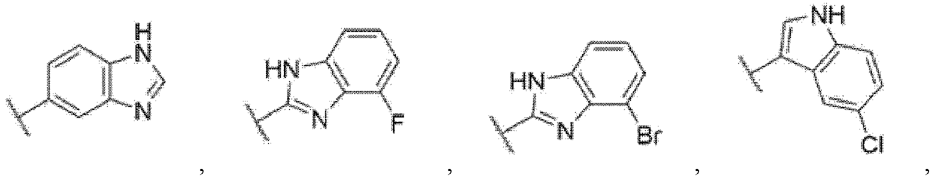

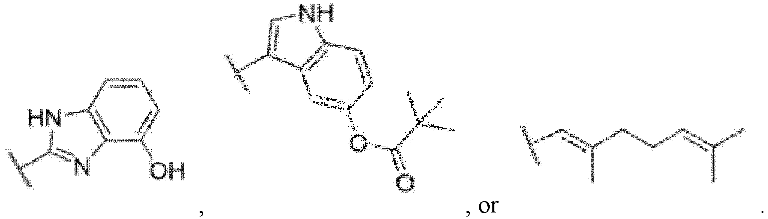

In one embodiment, the R² and R³ may each independently be -H, halogen, C₁₋₅alkyl, C₁₋₄alkyl, C₁₋₃alkyl, C₁₋₂alkyl, or -CH₃.

In one embodiment, the L¹ may be a bond, C₁₋₅alkylene, C₁₋₄alkylene, C₁₋₃alkylene, or C₁₋₂alkylene.

In one embodiment, the R⁴ may be C₁₋₅alkyl, C₁₋₄alkyl, C₁₋₃alkyl, C₁₋₂alkyl, -CH₃, C₃₋₅alkyl substituted with 1 or more, 2 or more, 3 to 5, or 4 -OH groups. Alternatively, specifically, the R⁴ may be

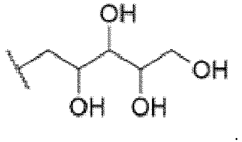

In one embodiment, the compound represented by the Chemical Formula 1 may be a compound represented by the following Chemical Formula 2:

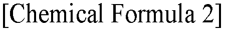

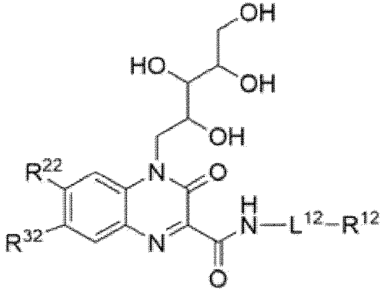

At this time, R¹, R², R³ and L¹ defined above can be equally applied to the R¹², R²², R³² and L¹².
Specifically, for example, R¹² is unsubstituted or substituted C₅₋₈ cycloalkyl, unsubstituted or substituted C₆₋₁₀aryl, unsubstituted or substituted heteroaryl of 5 to 8 atoms containing one or more heteroatoms selected from the group consisting of N, O and S, unsubstituted or substituted fused heteroaryl of 8 to 10 atoms containing one or more heteroatoms selected from the group consisting of N, O and S, or C₁₋₁₅alkenyl,
wherein the substituted Cs-scycloalkyl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₁₋₈alkoxycarbonyl, C₁₋₈alkoxycarbonylC₁₋₅alkyl, and C₁₋₈alkylcarbonyloxy,
the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₆₋₁₀aryl, C₁₋₈alkoxycarbonyl, C₁₋₈alkoxycarbonylC₁₋₅alkyl, C₁₋₈alkylcarbonyloxy, and heteroaryl of 5 to 8 atoms containing one or more heteroatoms selected from the group consisting of N, O and S,
the substituted heteroaryl of 5 to 8 atoms is substituted with halogen, or C₆₋₁₀aryl substituted with halogen, and
the substituted fused heteroaryl of 8 to 10 atoms is substituted with one or more substituents selected from the group consisting of -OH, halogen, C₁₋₈alkoxycarbonyl, C₁₋₈alkoxycarbonylC₁₋₅alkyl, and C₁₋₈alkylcarbonyloxy;
R²² and R³² are each independently -H, halogen, or C₁₋₁₀alkyl; and
L¹² may be a bond, or C₁₋₁₀alkylene.

One embodiment may include all stereoisomers of the compound represented by the Chemical Formula 2. For example, the compound represented by the Chemical Formula 2 may include a stereoisomeric compound represented by the following Chemical Formula 2A. However, the following Chemical Formula 2A only describes one example of various stereoisomeric compounds, and is not necessarily limited to the following compound.

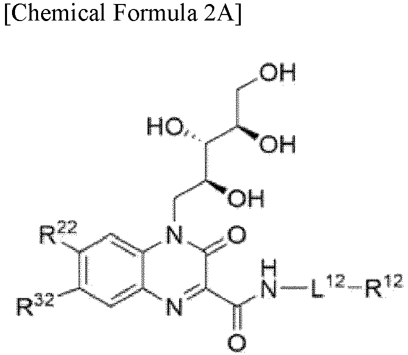

In one embodiment, the compound represented by the Chemical Formula 1 may be a compound represented by the following Chemical Formula 3.

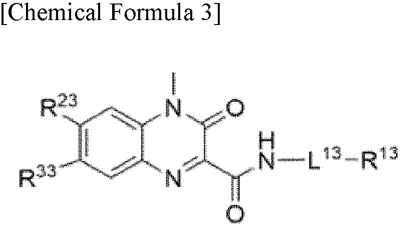

At this time, R¹, R², R³ and L¹ defined above may be equally applied to the R¹³, R²³, R³³ and L¹³.

Specifically, for example, R¹³ is unsubstituted or substituted C₆₋₁₀aryl or unsubstituted or substituted fused heteroaryl of 8 to 10 atoms containing one or more heteroatoms selected from the group consisting of N, O and S,
wherein the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀haloalkyl, C₁₋₈alkoxycarbonyl, C₁₋₈alkoxycarbonylC₁₋₅alkyl, and C₁₋₈alkylcarbonyloxy, and
the substituted fused heteroaryl of 8 to 10 atoms is substituted with one or more substituents selected from the group consisting of -OH and halogen;
R²³ and R³³ are each independently -H, halogen, or C₁₋₁₀alkyl; and
L¹³ may be a bond, or C₁₋₁₀alkylene.

In one embodiment, the compound represented by the Chemical Formula 1 may be any one selected from the following group of compounds.
(1) N-(4-methoxybenzyl)-6,7-dimethyl-3-oxo-4-((2S,3S,4R)-2,3,4,5-tetrahydroxypentyl)-3,4-dihydroquinoxaline-2-carboxamide;
(2) 6,7-dimethyl-N-(4-methylbenzyl)-3-oxo-4-((2S,3S,4R)-2,3,4,5-tetrahydroxypentyl)-3,4-dihydroquinoxaline-2-carboxamide;
(3) 6,7-dimethyl-3-oxo-4-((2S,3S,4R)-2,3,4,5-tetrahydroxypentyl)-N-(4-(trifluoromethyl)benzyl)- 3,4-dihydroquinoxaline-2-carboxamide;
(4) N-(4-fluorobenzyl)-6,7-dimethyl-3-oxo-4-((2S,3S,4R)-2,3,4,5-tetrahydroxypentyl)-3 ,4-dihydroquinoxaline-2-carboxamide;
(5) 6,7-dimethyl-3-oxo-4-((2S,3S,4R)-2,3,4,5-tetrahydroxypentyl)-N-(3-(trifluoromethyl) Benzyl)-3,4-dihydroquinoxaline-2-carboxamide;
(6) N-([1,1'-biphenyl]-4-ylmethyl)-6,7-dimethyl-3-oxo-4-((2S,3S,4R)-2,3,4,5-tetrahydroxypentyl)-3,4-dihydroquinoxaline-2-carboxamide;
(7) N-(3,5-dichlorobenzyl)-6,7-dimethyl-3-oxo-4-((2S,3S,4R)-2,3,4,5-tetrahydroxypentyl)-3,4-dihydroquinoxaline-2-carboxamide;
(8) N-(3,4-dichlorobenzyl)-6,7-dimethyl-3-oxo-4-((2S,3S,4R)-2,3,4,5-tetrahydroxypentyl)-3,4-dihydroquinoxaline-2-carboxamide;
(9) N-(2,4-dichlorophenethyl)-6,7-dimethyl-3-oxo-4-((2S,3S,4R)-2,3,4,5-tetrahydroxypentyl )-3,4-dihydroquinoxaline-2-carboxamide;
(10) N-(3-(4-bromophenyl)isoxazol-5-yl)-6,7-dimethyl-3-oxo-4-((2S,3S,4R)-2,3,4,5-tetrahydroxypentyl)-3,4-dihydroquinoxaline-2-carboxamide;
(11) N-(5-(4-bromophenyl)-1,3,4-thiadiazol-2-yl)-6,7-dimethyl-3-oxo-4-((2S,3S ,4R)-2,3,4,5-tetrahydroxypentyl)-3,4-dihydroquinoxaline-2-carboxamide;
(12) N-(3-(4-bromophenyl)-1H-pyrazol-5-yl)-6,7-dimethyl-3-oxo-4-((2S,3S,4R)-2,3,4,5-tetrahydroxypentyl)-3,4-dihydroquinoxaline-2-carboxamide;
(13) N-(4-(4-bromophenyl)thiazol-2-yl)-6,7-dimethyl-3-oxo-4-((2S,3S,4R)-2,3,4,5-tetrahydroxypentyl)-3,4-dihydroquinoxaline-2-carboxamide;
(14) N-((E)-3,7-dimethylocta-2,6-dien-1-yl)-6,7-dimethyl-3-oxo-4-((2S,3S,4R)-2,3,4,5-tetrahydroxypentyl)-3,4-dihydroquinoxaline-2-carboxamide;
(15) Methyl 2-(4-((6,7-dimethyl-3-oxo-4-((2S,3S,4R)-2,3,4,5-tetrahydroxypentyl)-3,4 -dihydroquinoxaline-2-carboxamido)methyl)phenyl)acetate;
(16) Methyl 3-((6,7-dimethyl-3-oxo-4-((2S,3S,4R)-2,3,4,5-tetrahydroxypentyl)-3,4-dihydroquinoxaline-2-carboxamido)methyl)benzoate;
(17) N-(2-(1H-benzo[d]imidazol-2-yl)ethyl)-6,7-dimethyl-3-oxo-4-((2S,3S,4R)-2,3,4,5-tetrahydroxypentyl)-3,4-dihydroquinoxaline-2-carboxamide;
(18) Methyl 4-((6,7-dimethyl-3-oxo-4-((2S,3S,4R)-2,3,4,5-tetrahydroxypentyl)-3,4-dihydroquinoxaline-2-carboxamido)methyl)benzoate;
(19) Methyl 2-(3-(6,7-dimethyl-3-oxo-4-((2S,3S,4R)-2,3,4,5-tetrahydroxypentyl)-3,4-dihydroquinoxaline-2-carboxamido)phenyl)acetate;
(20) Methyl 4-(6,7-dimethyl-3-oxo-4-((2S,3S,4R)-2,3,4,5-tetrahydroxypentyl)-3,4-dihydroquinoxaline-2-carboxamido)benzoate;
(21) N-(3-(1H-benzo[d]imidazol-2-yl)propyl)-6,7-dimethyl-3-oxo-4-((2S,3S,4R)-2,3,4,5-tetrahydroxypentyl)-3,4-dihydroquinoxaline-2-carboxamide;
(22) N-((1H-indol-6-yl)methyl)-6,7-dimethyl-3-oxo-4-((2S,3S,4R)-2,3,4,5-tetrahydroxypentyl)-3,4-dihydroquinoxaline-2-carboxamide;
(23) N-(4-(1H-imidazol-1-yl)phenyl)-6,7-dimethyl-3-oxo-4-((2S,3S,4R)-2,3,4,5-tetrahydroxypentyl)-3,4-dihydroquinoxaline-2-carboxamide;
(24) N-((1H-benzo[d]imidazol-2-yl)methyl)-6,7-dimethyl-3-oxo-4-((2S,3S,4R)-2,3,4,5-tetrahydroxypentyl)-3,4-dihydroquinoxaline-2-carboxamide;
(25)N-(3-1H-imidazol-1-yl)propyl)-6,7-dimethyl-3-oxo-4-((2S,3S,4R)-2,3,4,5-tetrahydroxypentyl)-3,4-dihydroquinoxaline-2-carboxamide;
(26) N-(3-(1H-indol-1-yl)propyl)-6,7-dimethyl-3-oxo-4-((2S,3S,4R)-2,3,4,5-tetrahydroxypentyl)-3,4-dihydroquinoxaline-2-carboxamide;
(27) N-(2-(5-hydroxy-1H-indol-3-yl)ethyl)-6,7-dimethyl-3-oxo-4-((2S,3S,4R)-2,3,4,5-tetrahydroxypentyl)-3,4-dihydroquinoxaline-2-carboxamide;
(28) N-(3-(1H-benzo[d]imidazol-1-yl)propyl)-6,7-dimethyl-3-oxo-4-((2S,3S,4R)-2,3,4,5-tetrahydroxypentyl)-3,4,-dihydroquinoxaline-2-carboxamide;
(29) N-(2-(1H-indol-2-yl)ethyl)-6,7-dimethyl-3-oxo-4-((2S,3S,4R)-2,3,4,5-tetrahydroxypentyl)-3,4-dihydroquinoxaline-2-carboxamide;
(30) Methyl 3-(6,7-dimethyl-3-oxo-4-((2S,3S,4R)-2,3,4,5-tetrahydroxypentyl)-3,4-dihydroquinoxaline-2-carboxamido)-4-methylbenzoate;
(31) N-(3-(1H-indol-3-yl)propyl)-6,7-dimethyl-3-oxo-4-((2S,3S,4R)-2,3,4,5-tetrahydroxypentyl)-3,4-dihydroquinoxaline-2-carboxamide;
(32) N-(1H-indol-5-yl)-6,7-dimethyl-3-oxo-4-((2S,3S,4R)-2,3,4,5-tetrahydroxypentyl)-3,4-dihydroquinoxaline-2-carboxamide;
(33) Methyl 3-(6,7-dimethyl-3-oxo-4-((2S,3S,4R)-2,3,4,5-tetrahydroxypentyl)-3,4-dihydroquinoxaline-2-carboxamido)cyclopentane-1-carboxylate;
(34) N-((1H-benzo[d]imidazol-5-yl)methyl)-6,7-dimethyl-3-oxo-4-((2S,3S,4R)-2,3,4,5-tetrahydroxypentyl)-3,4-dihydroquinoxaline-2-carboxamide;
(35) N-(4-fluoro-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide;
(36) N-(4-bromo-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide;
(37) N-(2-(5-chloro-1H-indol-3-yl)ethyl)-6,7-dimethyl-3-oxo-4-((2S,3S,4R)-2,3,4,5-tetrahydroxypentyl)-3,4-dihydroquinoxaline-2-carboxamide;
(38) N-(2-(4-fluoro-1H-benzo[d]imidazol-2-yl)ethyl)-6,7-dimethyl-3-oxo-4-((2S,3S,4R)-2,3,4,5-tetrahydroxypentyl)-3,4-dihydroquinoxaline-2-carboxamide;
(39) N-(4-hydroxy-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide;
(40) 3-(2-(6,7-dimethyl-3-oxo-4-((2S,3S,4R)-2,3,4,5-tetrahydroxypentyl)-3,4-dihydroquinoxaline-2-carboxamido)ethyl)-1H-indol-5-yl pivalate;
(41) N-(2-(5-hydroxy-1H-indol-3-yl)ethyl)-4,6,7-trimethyl-3-oxo-3,4-dihydroquinoxaline-2-carboxamide;
(42) N-(2-(4-fluoro-1H-benzo[d]imidazol-2-yl)ethyl)-4,6,7-trimethyl-3-oxo-3,4-dihydroquinoxaline-2-carboxamide;
(43) N-(2-(5-chloro-1H-indol-3-yl)ethyl)-4,6,7-trimethyl-3-oxo-3,4-dihydroquinoxaline-2-carboxamide;
(44) methyl 4-methyl-3-(4,6,7-trimethyl-3-oxo-3,4-dihydroquinoxaline-2-carboxamido)benzoate;
(45) N-(5-(hydroxycarbamoyl)-2-methylphenyl)-4,6,7-trimethyl-3-oxo-3,4-dihydroquinoxaline-2-carboxamide;
(46) N-(3-(2-(hydroxyamino)-2-oxoethyl)phenyl)-6,7-dimethyl-3-oxo-4-((2S,3S,4R)-2,3,4,5-tetrahydroxypentyl)-3,4-dihydroquinoxaline-2-carboxamide;
(47) N-(4-(hydroxycarbamoyl)benzyl)-6,7-dimethyl-3-oxo-4-((2S,3S,4R)-2,3,4,5-tetrahydroxypentyl)-3,4-dihydroquinoxaline-2-carboxamide;
(48) N-(4-(hydroxycarbamoyl)phenyl)-6,7-dimethyl-3-oxo-4-((2S,3S,4R)-2,3,4,5-tetrahydroxypentyl)-3,4-dihydroquinoxaline-2-carboxamide;
(49) N-(4-(2-(hydroxyamino)-2-oxoethyl)benzyl)-6,7-dimethyl-3-oxo-4-((2S,3S,4R)-2,3,4,5-tetrahydroxypentyl)-3,4-dihydroquinoxaline-2-carboxamide;
(50) N-(3-(hydroxycarbamoyl)benzyl)-6,7-dimethyl-3-oxo-4-((2S,3S,4R)-2,3,4,5-tetrahydroxypentyl)-3,4-dihydroquinoxaline-2-carboxamide;
(51) N-(5-(hydroxycarbamoyl)-2-methylphenyl)-6,7-dimethyl-3-oxo-4-((2S,3S,4R)-2,3,4,5- tetrahydroxypentyl)-3,4-dihydroquinoxaline-2-carboxamide; and
(52) N-(3-(hydroxycarbamoyl)cyclopentyl)-6,7-dimethyl-3-oxo-4-((2S,3S,4R)-2,3,4,5-tetrahydroxypentyl)-3,4-dihydroquinoxaline-2-carboxamide.

In one embodiment, the compound represented by Chemical Formula 1 can be used in the form of a pharmaceutically acceptable salt, and an acid addition salt formed by a pharmaceutically acceptable free acid is useful as the salt. Acid addition salts are obtained from inorganic acids such as hydrochloric acid, nitric acid, phosphoric acid, sulfuric acid, hydrobromic acid, hydroiodic acid, nitrous acid, phosphorous acid, etc., non-toxic organic acids such as aliphatic mono and dicarboxylates, phenyl-substituted alkanoates, hydroxy alkanoates and alkanedioates, aromatic acids, aliphatic and aromatic sulfonic acids, etc., organic acids such as trifluoroacetic acid, acetate, benzoic acid, citric acid, lactic acid, maleic acid, gluconic acid, methanesulfonic acid, 4-toluenesulfonic acid, tartaric acid, fumaric acid, etc. These types of pharmaceutically non-toxic salts include sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methyl benzoate, dinitro benzoate, hydroxybenzoate, methoxybenzoate, phthalate, terephthalate, benzenesulfonate, toluenesulfonate, chlorobenzenesulfonate, xylene sulfonate, phenylacetate, phenylpropionate, phenylbutyrate, citrate, lactate, β-hydroxybutyrate, glycolate, malate, tartrate, methanesulfonate, propane sulfonate, naphthalene-1-sulfonate, naphthalene-2-sulfonate, mandelate etc.

One embodiment provides a method of preparing the compound represented by Chemical Formula 1.

A method of preparing a compound represented by Chemical Formula 1 according to an embodiment includes, as shown in the following Reaction Scheme 1,
reacting a compound represented by Chemical Formula 1A with a hydroxide ion (OH⁻) to prepare a compound represented by Chemical Formula 1B; and
reacting the compound represented by Chemical Formula 1B with a compound represented by Chemical Formula 1C to prepare a compound represented by Chemical Formula 1.

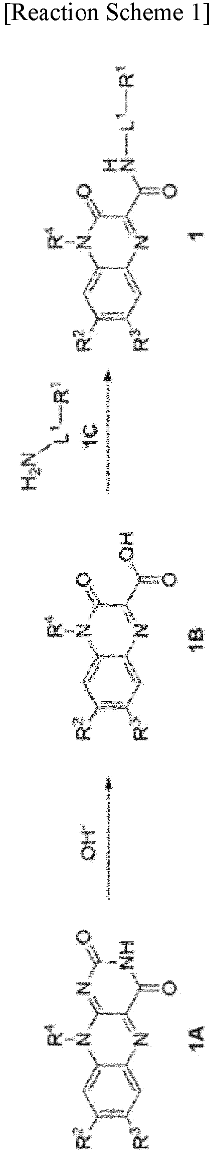

In the Reaction Scheme 1, the definitions above may be applied to R¹ to R⁴ and L¹. Therefore, the Chemical Formula 1 may be Chemical Formula 2 or Chemical Formula 3, and the R¹ to R³ and L¹ may be R¹² to R³² and L¹², or R¹³ to R³³ and L¹³.

The step of reacting with the hydroxide ion is a step of reacting the compound represented by Chemical Formula 1A with a compound containing the hydroxide ion, and for example, basic compounds such as NaOH, KOH, Ba(OH)₂, Ca(OH)₂, NH₄OH, Mg(OH)₂, etc., or H₂O (hydrolysis reaction) may be used, but this is only an example and is not necessarily limited to the use of the above compound.

Another embodiment provides a pharmaceutical composition for preventing or ameliorating cancer disease, including the compound represented by Chemical Formula 1, a stereoisomer thereof, a hydrate thereof, or a pharmaceutically acceptable salt thereof as an active ingredient.

The cancer disease may be selected from the group consisting of prostate cancer, liver cancer, lung cancer, breast cancer, ovarian cancer, uterine cancer, pancreatic cancer, lung cancer, stomach cancer, colon cancer, skin cancer, head or neck cancer, brain cancer, larynx cancer, bladder cancer, esophagus cancer, thyroid cancer, kidney cancer, and rectal cancer.

In one embodiment, the pharmaceutical composition may selectively inhibit histone deacetylase 8 (HDAC8), and may exert a preventive or therapeutic effect on cancer disease by selectively inhibiting HDAC8. The pharmaceutical composition according to one embodiment has an excellent effect of selectively inhibiting HDAC8 among HDACs, thereby minimizing unwanted side effects, and is therefore useful as a replacement for compounds (drugs) currently known as HDAC8 inhibitors.

In one embodiment, the composition including the compound represented by Chemical Formula 1 or a pharmaceutically acceptable salt thereof is not limited to use in the treatment or prevention of cancer disease as well as other cancers in which the anticancer effect is exerted by selectively inhibiting HDAC8. Accordingly, the composition may be a pharmaceutical composition for preventing or treating HDAC8-related cancer, or may be an HDAC8 inhibitor.

The compound represented by Chemical Formula 1 or a pharmaceutically acceptable salt thereof may be administered in various oral and parenteral formulations during clinical administration. In the case of formulation, it is prepared using diluents or excipients such as fillers, extenders, binders, wetting agents, disintegrants, and surfactants that are conventionally used. Solid preparations for oral administration include tablets, pills, acids, granules, and capsules, etc., and such solid preparations may be prepared by mixing, in addition to one or more compounds, at least one or more excipients such as starch, calcium carbonate, sucrose, lactose, and gelatin. In addition to simple excipients, lubricants such as magnesium stearate and talc are also used. Liquid preparations for oral administration include suspensions, oral solutions, emulsions, syrups, etc., and in addition to the commonly used simple diluents such as water and liquid paraffin, various excipients such as wetting agents, sweeteners, aromatics, and preservatives, etc., may be included. Preparations for parenteral administration include sterilized aqueous solutions, non-aqueous solvents, suspensions, and emulsions. As non-aqueous solvents and suspensions, propylene glycol, polyethylene glycol, vegetable oils such as olive oil, and injectable ester such as ethyl oleate may be used.

A pharmaceutical composition including the compound represented by Chemical Formula 1 or a pharmaceutically acceptable salt thereof as an active ingredient can be administered parenterally, and parenteral administration is by subcutaneous injection, intravenous injection, intramuscular injection, or intrathoracic injection.

At this time, in order to formulate into a formulation for parenteral administration, the compound represented by Chemical Formula 1 or a pharmaceutically acceptable salt thereof is mixed with water along with a stabilizer or buffer to prepare a solution or suspension, which can be prepared in an ampoule or vial unit dosage form. The composition may be sterile and/or contain auxiliaries such as preservatives, stabilizers, wetting agents or emulsification accelerators, salts and/or buffers for adjusting osmotic pressure, and other therapeutically useful substances, and may be formulated according to conventional mixing, granulating or coating methods.

The formulations for oral administration include, for example, tablets, pills, hard/soft capsules, solutions, suspensions, emulsifiers, syrups, granules, elixirs, troches, etc., and in addition to the active ingredients, such formulations contain diluents (e.g., lactose, dextrose, sucrose, mannitol, sorbitol, cellulose and/or glycine) and lubricants (e.g., silica, talc, stearic acid and magnesium or calcium salts thereof and/or polyethylene glycol). The tablets may contain binders such as magnesium aluminum silicate, starch paste, gelatin, methylcellulose, sodium carboxymethylcellulose and/or polyvinylpyrrolidine, etc., and depending on cases, may contain disintegrants such as starch, agar, alginic acid or sodium salts thereof, etc., or effervescent mixtures and/or absorbents, colorants, flavoring agents, and sweeteners.

Another embodiment provides a selective inhibitor of HDAC8 including the compound represented by Chemical Formula 1, a stereoisomer thereof, a hydrate thereof, or a salt thereof as an active ingredient.

Another embodiment provides a health functional food composition for preventing or ameliorating cancer disease, including the compound represented by Chemical Formula 1, a stereoisomer thereof, a hydrate thereof, or a salt thereof as an active ingredient.

The compound represented by Chemical Formula 1 according to one embodiment may be added to food as is or used together with other foods or food ingredients, and may be used appropriately according to conventional methods. The mixing amount of the active ingredient may be appropriately determined depending on the purpose of use (prevention or amelioration). Generally, the amount of the compound in health food may be 0.1 to 90 parts by weight of the total weight of the food. However, in the case of long-term intake for health and hygiene purposes or health control, the amount may be below the above range, and since there is no problem in terms of safety, the active ingredient may be used in amounts exceeding the above range.

In addition, the health functional food composition according to one embodiment has no particular restrictions on other ingredients other than containing the above compound, and may contain various flavoring agents or natural carbohydrates as additional ingredients like ordinary beverages.

Furthermore, it may contain various nutrients, vitamins, minerals (electrolytes), flavoring agents such as synthetic flavoring agents and natural flavoring agents, colorants and fillers (cheese, chocolate, etc.), pectic acid and salts thereof, alginic acid and salts thereof, organic acids, protective colloidal thickeners, pH adjusters, stabilizers, preservatives, glycerin, alcohol, carbonating agents used in carbonated beverages, or the like.

One embodiment provides a method of treating cancer disease, including administering the compound represented by Chemical Formula 1, a stereoisomer thereof, a hydrate thereof, or a pharmaceutically acceptable salt thereof to an individual or subject in need thereof.

One embodiment provides the compound represented by Chemical Formula 1, a stereoisomer thereof, a hydrate thereof, or a pharmaceutically acceptable salt thereof for use in the treatment of cancer disease.

One embodiment provides a use of the compound, a stereoisomer thereof, a hydrate thereof, or a pharmaceutically acceptable salt thereof for use in the manufacture of a drug for treating cancer disease.

### Modes for Carrying Out the Invention

Hereinafter, examples and experimental examples will be described in detail below. However, the examples and experimental examples described below are merely illustrative, and the technology described in this specification is not limited thereto.

The compounds of Examples 1 to 40 were prepared according to the following Reaction Scheme A.

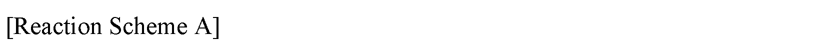

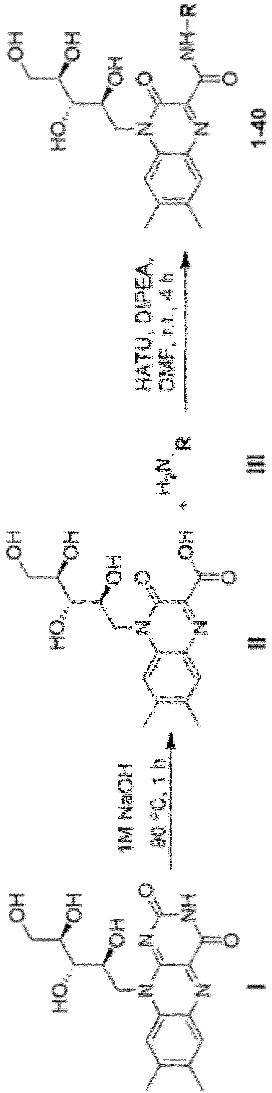

In the Reaction Scheme A, -R of compound III is a1 to a40 below.

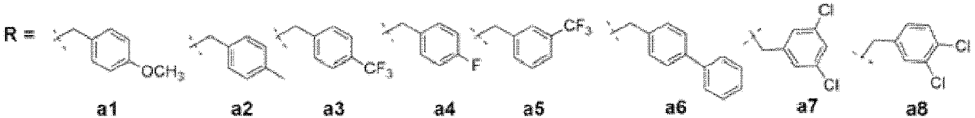

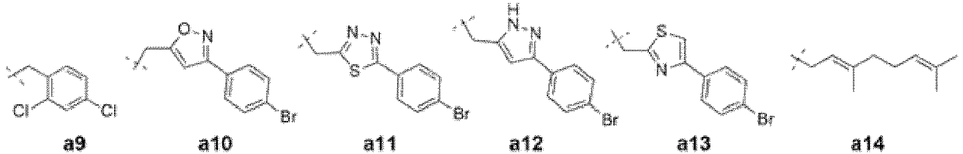

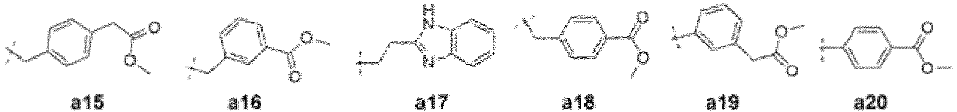

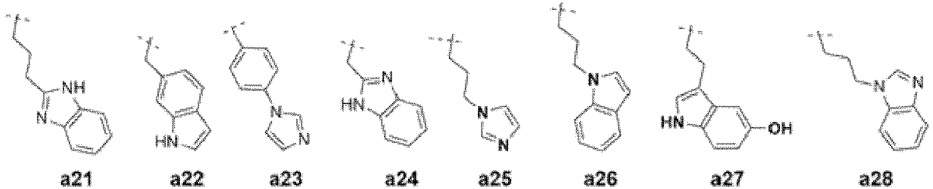

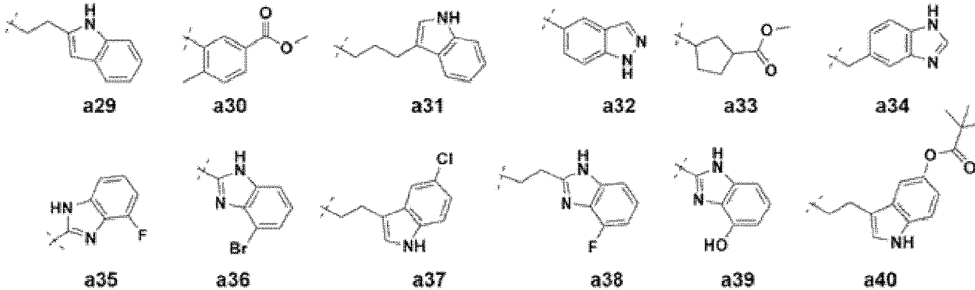

Hereinafter, specific methods for preparing Examples 1 to 40 are described.

### <Example 1> Preparation of N-(4-methoxybenzyl)-6,7-dimethyl-3-oxo-4-((2S,3S,4R)-2,3,4,5-tetrahydroxypentyl)-3,4-dihydroquinoxaline-2-carboxamide

After adding an equivalent amount of arylamine (III, R=a1) to the 6,7-dimethyl-3-oxo-4-((2S,3S,4R)-2,3,4,5-tetrahydroxypentyl)-3,4-dihydroquinoxaline-2-carboxylic acid (II) mixture in DMF solvent, 1.2 equivalents of 2-(3H-[1,2,3]triazole[4,5-b]pyridin-3-yl)-1,1,3,3-tetramethylisouranium hexafluorophosphate (HATU) and 5 equivalents of N,N-diisopropylethylamine (DIPEA) were slowly added and stirred at room temperature for 4 hours. The resulting mixture was purified using preparative HPLC (solvent system: acetonitrile, water) to obtain the compound of Example 1 as a yellow solid (15 mg, 37.4% yield).

ESI LC/MS: m/z calcd. for: C₂₄H₂₉N₃O₇ [M+H]⁺: 472.71; found 472.25. ¹H NMR (CD₃OD, 400MHz) *δ* 7.74 (s, 1H), 7.63 (s, 1H), 7.32 (d, *J =* 8.56 Hz, 2H), 6.89 (d, *J =* 8.64 Hz, 2H), 4.74 (q, *J =* 9.92 Hz, 1H), 4.58 (s, 2H), 4.41 (d, *J =* 13.96 Hz, 1H), 4.27-4.24 (m, 1H), 3.82-3.76 (m, 6H), 3.68-3.64 (m, 1H), 2.45 (s, 3H), 2.36 (s, 3H).

### <Example 2> Preparation of 6,7-dimethyl-N-(4-methylbenzyl)-3-oxo-4-((2S,3S,4R)-2,3,4,5-tetrahydroxypentyl)-3,4-dihydroquinoxaline-2-carboxamide

The compound of Example 2 as a yellow solid was obtained in the same manner as in Example 1, except that aryl amine (III, R = a2) was used instead of aryl amine (III, R = a1) (6 mg, 23.2% yield).

ESI LC/MS: m/z calcd. for: C₂₄H₂₉N₃O₆ [M+H]⁺: 456.21; found 456.01. ¹H NMR (CD₃OD, 400MHz) *δ* 7.74 (s, 1H), 7.63 (s, 1H), 7.28 (d, *J =* 7.84 Hz, 2H), 7.15 (d, *J =* 7.8 Hz, 2H), 4.74 (q, *J =* 9.96 Hz, 1H), 4.60 (s, 2H), 4.41 (d, *J =* 13.92 Hz, 1H), 4.27-4.24 (m, 1H), 3.83-3.74 (m, 3H), 3.69-3.64 (m, 1H), 2.44 (s, 3H), 2.36 (s, 3H), 2.31 (s, 3H).

### <Example 3> Preparation of 6,7-dimethyl-3-oxo-4-((2S,3S,4R)-2,3,4,5-tetrahydroxypentyl)-N-(4-(trifluoromethyl)benzyl)-3,4-dihydroquinoxaline-2-carboxamide

The compound of Example 3 as a yellow solid was obtained in the same manner as in Example 1, except that aryl amine (III, R = a3) was used instead of aryl amine (III, R = a1) (7 mg, 24.2% yield).

ESI LC/MS: m/z calcd. for: C₂₄H₂₇F₃N₃O₆ [M+H]⁺: 510.19; found 510.20. ¹H NMR (CD₃OD, 400MHz) *δ* 7.97 (s, 2H), 7.76 (s, 1H), 7.67-7.59 (m, 3H), 4.82-4.78 (m, 1H), 4.75 (s, 2H), 4.45 (d, *J* = 13.96 Hz, 1H), 4.29-4.26 (m, 1H), 3.83-3.75 (m, 3H), 3.69-3.65 (m, 1H), 2.46 (s, 3H), 2.38 (s, 3H).

### <Example 4> Preparation of N-(4-fluorobenzyl)-6,7-dimethyl-3-oxo-4-((2S,3S,4R)-2,3,4,5-tetrahydroxypentyl)-3,4-dihydroquinoxaline-2-carboxamide

The compound of Example 4 as a yellow solid was obtained in the same manner as in Example 1, except that aryl amine (III, R = a4) was used instead of aryl amine (III, R = a1) (8 mg, 30.7% yield).

ESI LC/MS: m/z calcd. for: C₂₃H₂₇FN₃O₆ [M+H]⁺: 460.19; found 460.20. ¹H NMR (CD₃OD, 400MHz) *δ* 7.77 (s, 1H), 7.66 (s, 1H), 7.45-7.41 (m, 2H), 7.08-7.04 (m, 2H), 4.76 (q, *J =* 10.52 Hz, 1H), 4.64 (s, 2H), 4.43 (d, *J =* 12.68 Hz, 1H), 4.27-4.25 (m, 1H), 3.82-3.75 (m, 3H), 3.69-3.65 (m, 1H), 2.46 (s, 3H), 2.38 (s, 3H).

### <Example 5> Preparation of (6,7-dimethyl-3-oxo-4-((2S,3S,4R)-2,3,4,5-tetrahydroxypentyl)-N-(3-(trifluoromethyl)benzyl)-3,4-dihydroquinoxaline-2-carboxamide

The compound of Example 5 as a yellow solid was obtained in the same manner as in Example 1, except that aryl amine (III, R = a5) was used instead of aryl amine (III, R = a1) (11 mg, 24.2% yield).

ESI LC/MS: m/z calcd. for: C₂₄H₂₇F₃N₃O₆ [M+H]⁺: 510.19; found 510.20. ¹H NMR (CD₃OD, 400MHz) *δ* 7.75-7.73 (m, 2H), 7.69 (d, *J =* 7.24 Hz, 1H), 7.65 (s, 1H), 7.59-7.52 (m, 2H), 4.81-4.78 (m, 1H), 4.73 (s, 2H), 4.43 (d, *J=* 13.88 Hz, 1H), 4.28-4.26 (m, 1H), 3.83-3.77 (m, 3H), 3.69-3.65 (m, 1H), 2.46 (s, 3H), 2.37 (s, 3H).

### <Example 6> Preparation of N-([1,1'-biphenyl]-4-ylmethyl)-6,7-dimethyl-3-oxo-4-((2S,3S,4R)-2,3,4,5-tetrahydroxypentyl)-3,4-dihydroquinoxaline-2-carboxamide

The compound of Example 6 as a yellow solid was obtained in the same manner as in Example 1, except that aryl amine (III, R = a6) was used instead of aryl amine (III, R = a1) (5 mg, 22.7% yield).

ESI LC/MS: m/z calcd. for: C₂₉H₃₂N₃O₆ [M+H]⁺: 518.23; found 518.20. ¹H NMR (CD₃OD, 400MHz) *δ* 7.78 (s, 1H), 7.67 (s, 1H), 7.60-7.58 (m, 4H), 7.48 (d, *J =* 8.04 Hz, 2H), 7.42 (t, *J =* 7.4 Hz, 2H), 7.32 (t, *J =* 7.32 Hz, 1H), 4.79 (q, *J =* 9.96 Hz, 1H), 4.71 (s, 2H), 4.45 (d, *J =* 13.88 Hz, 1H), 4.30-4.26 (m, 1H), 3.83-3.75 (m, 3H), 3.69-3.64 (m, 1H), 2.47 (s, 3H), 2.38 (s, 3H).

### <Example 7> Preparation of N-(3,5-dichlorobenzyl)-6,7-dimethyl-3-oxo-4-((2S,3S,4R)-2,3,4,5-tetrahydroxypentyl)-3,4-dihydroquinoxaline-2-carboxamide

The compound of Example 7 as a yellow solid was obtained in the same manner as in Example 1, except that aryl amine (III, R = a7) was used instead of aryl amine (III, R = a1) (6 mg, 27.6% yield).

ESI LC/MS: m/z calcd. for: C₂₃H₂₆Cl₂N₃O₆ [M+H]⁺: 510.12; found 510.15. ¹H NMR (CD₃OD, 400MHz) *δ* 7.77 (s, 1H), 7.68 (s, 1H), 7.38-7.33 (m, 3H), 4.80 (q, *J =* 10.96 Hz, 1H), 4.63 (s, 2H), 4.44 (d, *J =* 13.2 Hz, 1H), 4.29 (br, 1H), 3.83-3.78 (m, 3H), 3.68-3.65 (m, 1H), 2.47 (s, 3H), 2.38 (s, 3H).

### <Example 8> Preparation of N-(3,4-dichlorobenzyl)-6,7-dimethyl-3-oxo-4-((2S,3S,4R)-2,3,4,5-tetrahydroxypentyl)-3,4-dihydroquinoxaline-2-carboxamide

The compound of Example 8 as a yellow solid was obtained in the same manner as in Example 1, except that aryl amine (III, R = a8) was used instead of aryl amine (III, R = a1) (11 mg, 29.4% yield).

ESI LC/MS: m/z calcd. for: C₂₃H₂₆Cl₂N₃O₆ [M+H]⁺: 510.12; found 510.15. ¹H NMR (CD₃OD, 400MHz) *δ* 7.77 (s, 1H), 7.67 (s, 1H), 7.58 (s, 1H), 7.48 (d, *J* = 8.28 Hz, 1H), 7.34 (d, *J =* 8.24 Hz, 1H), 4.79 (q, *J =* 9.92 Hz, 1H), 4.63 (s, 2H), 4.44 (d, *J =* 13.92 Hz, 1H), 4.30-4.26 (m, 1H), 3.83-3.75 (m, 3H), 3.70-3.65 (m, 1H), 2.47 (s, 3H), 2.38 (s, 3H).

### <Example 9> Preparation of N-(2,4-dichlorophenethyl)-6,7-dimethyl-3-oxo-4-((2S,3S,4R)-2,3,4,5-tetrahydroxypentyl)-3,4-dihydroquinoxaline-2-carboxamide

The compound of Example 9 as a yellow solid was obtained in the same manner as in Example 1, except that aryl amine (III, R = a9) was used instead of aryl amine (III, R = a1) (8 mg, 26.9% yield).

ESI LC/MS: m/z calcd. for: C₂₄H₂₈Cl₂N₃O₆ [M+H]⁺: 524.14; found 524.15. ¹H NMR (CD₃OD, 400MHz) *δ* 7.78 (s, 1H), 7.68 (s, 1H), 7.44 (s, 1H), 7.38 (d, *J=* 8.24 Hz, 1H), 7.27 (d, *J* = 8.16 Hz, 1H), 4.79 (q, J= 10.28 Hz, 1H), 4.46 (d, *J =* 13.68 Hz, 1H), 4.28-4.25 (m. 1H), 3.83-3.67 (s, 6H), 3.11-3.08 (m, 2H), 2.48 (s, 3H), 2.39 (s, 3H).

### <Example 10> Preparation of N-(3-(4-bromophenyl)isoxazol-5-yl)-6,7-dimethyl-3-oxo-4-((2S,3S,4R)-2,3,4,5-tetrahydroxypentyl)-3,4-dihydroquinoxaline-2-carboxamide

The compound of Example 10 as a yellow solid was obtained in the same manner as in Example 1, except that aryl amine (III, R = a10) was used instead of aryl amine (III, R = a1) (6 mg, 18.4% yield).

ESI LC/MS: m/z calcd. for: C₂₅H₂₆BrN₄O₇ [M+H]⁺: 573.10; found 574.99. ¹H NMR (CD₃OD, 400MHz) *δ* 7.84 (br, 1H), 7.82-7.80 (m, 2H), 7.75 (s, 1H), 7.71-7.69 (m, 2H), 7.48 (s, 1H), 4.56-4.53 (m, 1H), 4.34 (br, 1H), 3.85-3.80 (m, 4H), 3.72-3.67 (m, 1H), 2.78 (s, 3H), 2.41 (s, 3H).

### <Example 11> Preparation of N-(5-(4-bromophenyl)-1,3,4-thiadiazol-2-yl)-6,7-dimethyl-3-oxo-4-((2S,3S,4R)-2,3,4,5-tetrahydroxypentyl)-3,4-dihydroquinoxaline-2-carboxamide

The compound of Example 11 as a yellow solid was obtained in the same manner as in Example 1, except that aryl amine (III, R = a11) was used instead of aryl amine (III, R = a1) (10 mg, 29.9% yield).

ESI LC/MS: m/z calcd. for: C₂₄H₂₅BrN₅O₆S[M+H]⁺: 590.06; found 591.90. ¹H NMR (CD₃OD, 400MHz) *δ* 7.88 (d, *J =* 8.52 Hz, 2H), 7.82 (s, 1H), 7.75 (s, 1H), 7.70 (d, *J =* 8.56 Hz, 2H), 4.59-4.55 (m, 1H), 4.37-4.34 (m, 1H), 3.86-3.81 (m, 3H), 3.73-3.69 (m, 2H), 2.49 (s, 3H), 2.40 (s, 3H).

### <Example 12> Preparation of N-(3-(4-bromophenyl)-1H-pyrazol-5-yl)-6,7-dimethyl-3-oxo-4-((2S,3S,4R)-2,3,4,5-tetrahydroxypentyl)-3,4-dihydroquinoxaline-2-carboxamide

The compound of Example 12 as a yellow solid was obtained in the same manner as in Example 1, except that aryl amine (III, R = a12) was used instead of aryl amine (III, R = a1) (15 mg, 23.1% yield).

ESI LC/MS: m/z calcd. for: C₂₅H₂₇BrN₅O₆[M+H]⁺: 572.11; found 573.95. ¹H NMR (DMSO-d₆, 400MHz) *δ* 7.74-7.71 (m, 3H), 7.66-7.64 (m, 3H), 7.06 (s, 1H), 4.66 (q, *J =* 10.2 Hz, 1H), 4.28 (d, *J =* 12 Hz, 1H), 4.15 (d, *J =* 9.44 Hz, 1H), 3.64-3.49 (m, 4H), 2.40 (s, 3H), 2.34 (s, 3H).

### <Example 13> Preparation of N-(4-(4-bromophenyl)thiazol-2-yl)-6,7-dimethyl-3-oxo-4-((2S,3S,4R)-2,3,4,5-tetrahydroxypentyl)-3,4-dihydroquinoxaline-2-carboxamide

The compound of Example 13 as a yellow solid was obtained in the same manner as in Example 1, except that aryl amine (III, R = a13) was used instead of aryl amine (III, R = a1) (6 mg, 17.9% yield).

ESI LC/MS: m/z calcd. for: C₂₅H₂₆BrN₄O₆S[M+H]⁺: 589.08; found 591.0. ¹H NMR (DMSO-d₆, 400MHz) *δ* 7.89 (d, *J =* 8.56 Hz, 2H), 7.85 (s, 1H), 7.75 (s, 1H), 7.69 (s, 1H), 7.62 (d, *J=* 8.6 Hz, 2H), 4.70-4.64 (m, 1H), 4.56-4.51 (m, 1H), 4.34-4.31 (m, 1H), 3.64-3.61 (m, 4H), 2.42 (s, 3H), 2.35 (s, 3H).

### <Example 14> Preparation of N-((E)-3,7-dimethylocta-2,6-dien-1-yl)-6,7-dimethyl-3-oxo-4-((2S,3S,4R)-2,3,4,5-tetrahydroxypentyl)-3,4-dihydroquinoxaline-2-carboxamide

The compound of Example 14 as a light-yellow solid was obtained in the same manner as in Example 1, except that aryl amine (III, R = a14) was used instead of aryl amine (III, R = a1) (32 mg, 66.5% yield).

ESI LC/MS: m/z calcd. for: C₂₆H₃₈N₃O₆ [M+H]⁺: 488.27; found 488.05. ¹H NMR (CD₃OD, 400MHz) *δ* 7.76 (s, 2H), 5.36 (s, 1H), 5.13 (s, 1H), 4.48 (m, 1H), 4.29 (s, 1H), 4.03 (s, 2H), 3.85 (m, 3H), 3.72 (m, 1H), 2 49 (s, 3H), 2.40 (s, 3H), 2.15 (m, 2H), 2.09 (m, 2H), 1.77 (s, 3H), 1.68 (s, 3H), 1.62 (s, 3H).

### <Example 15> Preparation of methyl 2-(4-((6,7-dimethyl-3-oxo-4-((2S,3S,4R)-2,3,4,5-tetrahydroxypentyl)-3,4-dihydroquinoxaline-2-carboxamido)methyl)phenyl)acetate

The compound of Example 15 as a yellow solid was obtained in the same manner as in Example 1, except that aryl amine (III, R = a15) was used instead of aryl amine (III, R = a1) (11 mg, 15.09% yield).

ESI LC/MS: m/z calcd. for: C₂₆H₃₁N₃O₈ [M+H]⁺: 514.54; found 514.21. ¹H NMR (CD₃OD, 400MHz) *δ* 7.79 (s, 1H), 7.68 (s, 1H), 7.38 (d, *J =* 8.0 Hz, 2H), 7.27 (d, *J =* 8.04 Hz, 2H), 4.77 (q, *J* = 9.92 Hz, 1H), 4.66 (s, 2H), 4.46 (dd, *J =* 14, 2.52 Hz, 1H), 4.30-4.26 (m, 1H), 3.85-3.76 (m, 3H), 3.71-3.69 (m, 1H), 3.68 (s, 3H), 3.65 (s, 2H), 2.48 (s, 3H), 2.39 (s, 3H).

### <Example 16> Preparation of methyl 3-((6,7-dimethyl-3-oxo-4-((2S,3S,4R)-2,3,4,5-tetrahydroxypentyl)-3,4-dihydroquinoxaline-2-carboxamido)methyl)benzoate

The compound of Example 16 as a yellow solid was obtained in the same manner as in Example 1, except that aryl amine (III, R = a16) was used instead of aryl amine (III, R = a1) (15 mg, 21.2% yield).

ESI LC/MS: m/z calcd. for: C₂₅H₂₉N₃O₈[M+H]⁺: 500.15; found 500.25. ¹H NMR (CD₃OD, 400MHz) *δ* 7.98 (s, 1H), 7.84 (d, *J =* 7.8 Hz, 1H), 7.70 (s, 1H), 7.59-7.58 (m, 2H), 7.38 (t, *J =* 7.76 Hz, 1H), 4.69 (q, *J =* 9.88 Hz, 1H), 4.66 (s, 2H), 4.37 (dd, *J =* 14.04, 2.64 Hz, 1H), 4.20-4.16 (m, 1H), 3.80 (s, 3H), 3.74-3.66 (m, 3H), 3.60-3.56 (m, 1H), 2.38 (s, 3H), 2.30 (s, 3H).

¹³C NMR (CD₃OD, 100MHz) *δ* 168.54, 157.13, 145.61, 145.05, 140.53, 135.55, 134.07, 133.74, 132.85, 132.07, 130.09, 129.91, 129.66, 117.37, 75.25, 74.36, 71.32, 64.99, 52.82, 46.47, 44.31, 21.03, 19.31.

### <Example 17> Preparation of N-(2-(1H-benzo[d]imidazol-2-yl)ethyl)-6,7-dimethyl-3-oxo-4-((2S,3S,4R)-2,3,4,5-tetrahydroxypentyl)-3,4-dihydroquinoxaline-2-carboxamide

The compound of Example 17 as a yellow solid was obtained in the same manner as in Example 1, except that aryl amine (III, R = a17) was used instead of aryl amine (III, R = a1) (118 mg, 55.9% yield).

ESI LC/MS: m/z calcd. for: C₂₅H₂₉N₅O₆ [M+H]⁺: 496.52; found 496.25. ¹H NMR (DMSO-d₆, 400MHz) *δ* 12.29 (s, 1H), 9.27 (t, *J =* 5.72 Hz, 1H), 7.64 (s, 1H), 7.60 (s, 1H), 7.54 (d, *J =* 7.76 Hz, 1H), 7.43 (d, *J =* 6.84 Hz, 1H), 7.15-7.09 (m, 2H), 4.98-4.81 (m, 2H), 4.66 (d, *J =* 6.2 Hz, 1-OH), 4.59 (q, *J =* 10.08 Hz, 1-OH), 4.48-4.46 (m, 1-OH), 4.22 (dd, *J =* 13.6, 2.08 Hz, 1-OH), 4.13-4.09 (m, 1H), 3.77 (q, *J =* 7.12 Hz, 2H), 3.64-3.59 (m, 3H), 3.45-3.41 (m, 1H), 3.10 (t, *J =* 7.2 Hz, 2H), 2.38 (s, 3H), 2.32 (s, 3H).

¹³C NMR (DMSO-d₆, 100MHz) *δ* 163.74, 154.42, 153.09, 148.41, 142.23, 133.10, 132.51, 130.89, 130.26, 122.04, 121.36, 118.68, 116.57, 111.32, 74.19, 73.12, 69.10, 63.89, 44.96, 37.75, 29.16, 20.76, 19.10.

### <Example 18> Preparation of methyl 4-((6,7-dimethyl-3-oxo-4-((2S,3S,4R)-2,3,4,5-tetrahydroxypentyl)-3,4-dihydroquinoxaline-2-carboxamido)methyl)benzoate

The compound of Example 18 as a light-yellow solid was obtained in the same manner as in Example 1, except that aryl amine (III, R = a18) was used instead of aryl amine (III, R = a1) (21 mg, 14.8% yield).

ESI LC/MS: m/z calcd. for: C₂₅H₂₉N₃O₈ [M+H]⁺: 500.51; found 500.15. ¹H NMR (CD₃OD, 400MHz) *δ* 8.01 (d, *J =* 6.88 Hz, 2H), 7.80 (s, 1H), 7.70 (s, 1H), 7.54 (t, *J =* 8.32 Hz, 1H), 4.78 (q, *J =* 9.84 Hz, 1H), 4.76 (s, 2H), 4.38 (dd, *J =* 14, 2.6 Hz, 1H), 4.32-4.27 (m, 1H), 3.91 (s, 3H), 3.85-3.77 (m, 3H), 3.72-3.67 (m, 1H), 2.49 (s, 3H), 2.40 (s, 3H).

¹³C NMR (CD₃OD, 100MHz) *δ* 166.93, 463.65, 144.05, 143.79, 143.55, 134.00, 132.47, 131.25, 130.47, 129.94, 128.94, 127.25, 115.79, 73.67, 72.78, 69.73, 63.41, 51.18, 44.87, 42.74, 19.44, 17.73.

### <Example 19> Preparation of methyl 2-(3-(6,7-dimethyl-3-oxo-4-((2S,3S,4R)-2,3,4,5-tetrahydroxypentyl)-3,4-dihydroquinoxaline-2-carboxamido)phenyl)acetate

The compound of Example 19 as a yellow solid was obtained in the same manner as in Example 1, except that aryl amine (III, R = a19) was used instead of aryl amine (III, R = a1) (112 mg, 52.57% yield).

ESI LC/MS: m/z calcd. for: C₂₅H₂₉N₃O₈ [M+H]⁺: 500.51; found 500.10. ¹H NMR (DMSO-d₆, 400MHz) *δ* 11.13 (s, 1H), 7.69 (s, 1H), 7.65-7.62 (m, 3H), 7.35-7.31 (m, 1H), 7.03 (d, *J =* 7.6 Hz, 1H), 4.99 (s, 1H), 4.84 (s, 1H), 4.69 (d, *J =* 6.28 Hz, 1H), 4.65 (q, *J=* 10.04 Hz, 1H), 4.49-4.47 (s, 1H), 4.26 (d, *J =* 13.68 Hz, 1H), 4.17-4.15 (m, 1H), 3.70 (s, 2H), 3.63-3.60 (m, 6H), 3.46-3.44 (m, 1H), 2.40 (s, 3H), 2.34 (s, 3H).

¹³C NMR (DMSO-d₆, 100MHz) *δ* 177.97, 162.23, 154.33, 148.92, 142.42, 138.94, 135.67, 133.26, 132.58, 130.88, 130.28, 129.48, 125.56, 120.81, 118.55, 116.66, 74.20, 73.15, 69.20, 63.91, 52.22, 45.02, 20.79, 19.11.

### <Example 20> Preparation of methyl 4-(6,7-dimethyl-3-oxo-4-((2S,3S,4R)-2,3,4,5-tetrahydroxypentyl)-3,4-dihydroquinoxaline-2-carboxamido)benzoate

The compound of Example 20 as a yellow solid was obtained in the same manner as in Example 1, except that aryl amine (III, R = a20) was used instead of aryl amine (III, R = a1) (32 mg, 46.6% yield).

ESI LC/MS: m/z calcd. for: C₂₄H₂₇N₃O₈ [M+H]⁺: 486.48; found 486.20. ¹H NMR (DMSO-d₆, 400MHz) *δ* 11.41 (s, 1H), 7.99 (d, *J =* 8.72 Hz, 2H), 7.86 (d, *J =* 8.76 Hz, 2H), 7.70 (s, 1H), 7.66 (s, 1H), 5.01 (s, 1H), 4.85 (s, 1H), 4.70 (d, *J =* 6.28 Hz, 1H), 4.65 (q, *J* = 10.12 Hz, 1H), 4.51-4.48 (m, 1H), 4.26 (d, *J =* 13.68 Hz, 1H), 4.17-4.15 (m, 1H), 3.86 (s, 3H), 3.65-3.61 (m, 3H), 3.46-3.42 (m, 1H), 2.41 (s, 3H), 2.34 (s, 3H).

¹³C NMR (DMSO-d₆, 100MHz) *δ* 177.22, 162.75, 154.19, 148.76, 143.17, 142.63, 133.35, 132.64, 130.96, 130.83, 130.29, 125.22, 119.54, 116.69, 74.19, 73.14, 69.19, 63.90, 52.45, 45.04, 20.81, 19.10.

### <Example 21> Preparation of N-(3-(1H-benzo[d]imidazol-2-yl)propyl)-6,7-dimethyl-3-oxo-4-((2S,3S,4R)-2,3,4,5-tetrahydroxypentyl)-3,4-dihydroquinoxaline-2-carboxamide

The compound of Example 21 as a light-yellow solid was obtained in the same manner as in Example 1, except that aryl amine (III, R = a21) was used instead of aryl amine (III, R = a1) (6 mg, 8.3% yield).

ESI LC/MS: m/z calcd. for: C₂₆H₃₁N₅O₆ [M+H]⁺: 510.55; found 510.20. ¹H NMR (CD₃OD, 400MHz) *δ* 7.75 (s, 1H), 7.69-7.65 (m, 3H), 7.46-7.43 (m, 2H), 4.79-7.63 (m, 2H), 4.46-4.40 (m, 1H), 4.28-4.25 (m, 1H), 3.87-3.79 (m, 2H), 3.73-3.65 (m, 3H), 3.01 (s, 1H), 2.88 (s, 1H), 2.50 (s, 3H), 2.42 (s, 3H), 2.36-2.32 (m, 2H).

### <Example 22> Preparation of N-((1H-indol-6-yl)methyl)-6,7-dimethyl-3-oxo-4-((2S,3S,4R)-2,3,4,5-tetrahydroxypentyl)-3,4-dihydroquinoxaline-2-carboxamide

The compound of Example 22 as a light-yellow solid was obtained in the same manner as in Example 1, except that aryl amine (III, R = a22) was used instead of aryl amine (III, R = a1) (10 mg, 14.67% yield).

ESI LC/MS: m/z calcd. for: C₂₅H₂₈N₄O₆ [M+H]⁺: 481.51; found 481.20. ¹H NMR (CD₃OD, 400MHz) *δ* 7.68 (s, 1H), 7.60 (m, 1H), 7.48 (d, *J =* 8.08 Hz, 1H), 7.47-7.34 (m, 2H), 7.22-7.21 (m, 1H), 7.04 (d, *J =* 8.12 Hz, 1H), 4.79-4.60 (m, 4H), 4.37 (dd, *J =* 13.88, 2.04 Hz, 1H), 4.27-4.18 (m, 2H), 3.83-3.65 (m, 6H), 2.42 (s, 3H), 2.33 (s, 3H).

### <Example 23> Preparation of N-(4-(1H-imidazol-1-yl)phenyl)-6,7-dimethyl-3-oxo-4-((2S,3S,4R)-2,3,4,5-tetrahydroxypentyl)-3,4-dihydroquinoxaline-2-carboxamide

The compound of Example 23 as a yellow solid was obtained in the same manner as in Example 1, except that aryl amine (III, R = a23) was used instead of aryl amine (III, R = a1) (34 mg, 48.5% yield).

ESI LC/MS: m/z calcd. for: C₂₅H₂₇N₅O₆ [M+H]⁺: 494.51; found 494.15. ¹H NMR (DMSO-d₆, 400MHz) *δ* 11.12 (s, 1H), 8.23 (s, 1H), 7.87-7.84 (m, 2H), 7.73-7.72 (m, 2H), 7.68-7.66 (m, 3H), 7.10 (s, 1H), 5.01 (s, 1H), 4.86 (s, 1H), 4.70 (d, *J* = 6.33 Hz, 1H), 4.66 (d, *J* = 13.67 Hz, 1H), 4.50-4.48 (m, 1H), 4.26 (dd, *J =* 13.76, 2.08 Hz, 1H), 4.18-4.15 (m, 1H), 3.66-3.62 (m, 3H), 3.47-3.44 (m, 1H), 2.41 (s, 3H), 2.34 (s, 3H).

¹³C NMR (DMSO-d₆, 100MHz) *δ* 162.34, 154.25, 148.82, 142.53, 137.58, 135.91, 133.31, 132.62, 130.85, 130.25, 121.19, 121.08, 118.49, 116.69, 74.20, 73.16, 69.19, 66.94, 63.91, 45.02, 36.24, 31.35, 20.80, 19.12.

### <Example 24> Preparation of N-((1H-benzo[d]imidazol-2-yl)methyl)-6,7-dimethyl-3-oxo-4-((2S,3S,4R)-2,3,4,5-tetrahydroxypentyl)-3,4-dihydroquinoxaline-2-carboxamide

The compound of Example 24 as a yellow solid was obtained in the same manner as in Example 1, except that aryl amine (III, R = a24) was used instead of aryl amine (III, R = a1) (7.8 mg, 14.3% yield).

ESI LC/MS: m/z calcd. for: C₂₄H₂₇N₅O₆ [M+H]⁺: 482.50; found 482.19. ¹H NMR (CD₃OD, 400MHz) *δ* 7.80 (s, 1H), 7.74 (s, 1H), 7.69-7.66 (m, 2H), 7.46-7.43 (m, 2H), 5.09 (s, 2H), 4.82 (s, 1H), 4.53 (dd, *J =* 14.04, 2.44 Hz, 1H), 4.34-4.30 (m, 1H), 3.86-3.79 (m, 3H), 3.73-3.68 (m, 1H), 2.51 (s, 3H), 2.41 (s, 3H).

### <Example 25> Preparation of N-(3-(1H-imidazol-1-yl)propyl)-6,7-dimethyl-3-oxo-4-((2S,3S,4R)-2,3,4,5-tetrahydroxypentyl)-3,4-dihydroquinoxaline-2-carboxamide

The compound of Example 25 as a yellow solid was obtained in the same manner as in Example 1, except that aryl amine (III, R = a25) was used instead of aryl amine (III, R = a1) (9 mg, 21.9% yield).

ESI LC/MS: m/z calcd. for: C₂₂H₂₉N₅O₆ [M+H]⁺: 460.22; found 460 ¹H NMR (MeOD, 400MHz) *δ* 8.00 (s, 1H), 7.79 (q, *J =* 12.24 Hz, 3H), 7.59 (s, 1H), 7.70 (d, *J =* 8.56 Hz, 2H), 4.43 (t, *J =* 6.8 Hz, 2H), 3.83-3.80 (m, 2H), 3.56-3.50 (m, 2H), 3.01 (s, 3H), 2.88 (s, 3H), 2.51 (s, 3H), 2.42 (s, 3H), 2.30-.2.28 (m, 2H).

### <Example 26> Preparation of N-(3-(1H-indol-1-yl)propyl)-6,7-dimethyl-3-oxo-4-((2S,3S,4R)-2,3,4,5-tetrahydroxypentyl)-3,4-dihydroquinoxaline-2-carboxamide

The compound of Example 26 as a yellow solid was obtained in the same manner as in Example 1, except that aryl amine (III, R = a26) was used instead of aryl amine (III, R = a1) (20 mg, 43.9% yield).

ESI LC/MS: m/z calcd. for: C₂₇H₃₂N₄O₆ [M+H]⁺: 509.24; found 509.30 ¹H NMR (MeOD, 400MHz) *δ* 7.78 (s, 1H), 7.70 (s, 1H), 7.52 (dd, *J =* 22.72, 7.88 Hz, 2H), 7.29 (d, *J* = 0.72 Hz, 1H), 7.16-7.12 (m, 1H), 7.01-6.97 (m, 1H), 6.44 (q, *J =* 0.72 Hz, 1H), 4.35 (t, *J =* 6.84 Hz, 3H), 3.84-3.83 (m, 3H), 3.82-3.80 (m, 3H), 3.75 (t, *J =* 6.6 Hz, 2H), 3.49 (q, *J* = 6.72 Hz, 2H), 2.83 (s, 2H), 2.50 (s, 3H), 2.41 (s, 3H).

### <Example 27> Preparation of N-(2-(5-hydroxy-1H-indol-3-yl)ethyl)-6,7-dimethyl-3-oxo-4-((2S,3S,4R)-2,3,4,5-tetrahydroxypentyl)-3,4-dihydroquinoxaline-2-carboxamide

The compound of Example 27 as a yellow solid was obtained in the same manner as in Example 1, except that aryl amine (III, R = a27) was used instead of aryl amine (III, R = a1) (8.5mg, 18.8% yield).

ESI LC/MS: m/z calcd. for: C₂₆H₃₀N₄O₇ [M+H]⁺: 511.21; found 511.27 ¹H NMR (MeOD, 400MHz) *δ* 7.81 (s, 1H), 7.70 (s, 1H), 7.18 (t, *J =* 8.4 Hz, 2H), 6.99 (s, 1H), 6.67(d, *J* = 7.96 Hz, 1H), 4.78 (d, *J* = 11.84 Hz, 1H), 4.49 (d, *J* = 12.52 Hz, 1H), 4.29 (s, 1H), 3.83-3.71 (m, 6H), 3.10-3.05 (m, 2H), 2.50 (s, 3H), 2.41 (s, 3H), 2.83 (s, 2H), 2.50 (s, 3H), 2.41 (s, 3H).

¹³C NMR (DMSO-d₆, 100MHz) *δ* 163.56, 154.42, 150.67, 148.65, 133.04, 132.50, 131.27, 130.90, 130.29, 128.29, 123.69, 116.55, 112.14, 111.77, 111.02, 102.69, 74.22, 73.13, 69.12, 63.90, 44.91, 25.64, 20.76, 19.12.

### <Example 28> Preparation of N-(3-(1H-benzo[d]imidazol-1-yl)propyl)-6,7-dimethyl-3-oxo-4-((2S,3S,4R)-2,3,4,5-tetrahydroxypentyl)-3,4-dihydroquinoxaline-2-carboxamide

The compound of Example 28 as a yellow solid was obtained in the same manner as in Example 1, except that aryl amine (III, R = a28) was used instead of aryl amine (III, R = a1) (13 mg, 29.8% yield).

ESI LC/MS: m/z calcd. for: C₂₆H₃₁N₅O₆ [M+H]⁺: 510.23; found 510.30 ¹H NMR (MeOD, 400MHz) *δ* 8.06 (d, *J =* 8.08 Hz, 1H), 7.86 (dd, *J =* 7.6, 2.2 Hz, 1H), 7.79 (s, 1H), 7.72-7.64 (m, 3H), 4.83 (d, *J =* 3.2 Hz, 1H), 4.80-4.67 (m, 3H), 4.65-4.60 (m, 1H), 4.58-4.47 (m, 1H), 4.33-4.30 (m, 1H), 3.84-3.81 (m, 2H), 3.62 (t, *J =* 6.16 Hz, 3H), 2.51 (s, 3H), 2.42 (s, 3H).

### <Example 29> Preparation of N-(2-(1H-indol-2-yl)ethyl)-6,7-dimethyl-3-oxo-4-((2S,3S,4R)-2,3,4,5-tetrahydroxypentyl)-3,4-dihydroquinoxaline-2-carboxamide

The compound of Example 29 as a yellow solid was obtained in the same manner as in Example 1, except that aryl amine (III, R = a29) was used instead of aryl amine (III, R = a1) (21 mg, 49.1% yield).

ESI LC/MS: m/z calcd. for: C₂₆H₃₀N₄O₇ [M+H]⁺: 495.26; found 495.22 ¹H NMR (MeOD, 400MHz) *δ* 7.79 (s, 1H), 7.69 (s, 1H), 7.45 (d, *J =* 7.76 Hz, 1H), 7.31 (d, *J =* 8.04 Hz 1H), 7.05-7.01 (m, 1H), 6.97 (q, *J =* 0.92 Hz, 1H), 4.49 (dd, *J =* 14.0, 2.48 Hz, 1H), 4.30-4.26 (m, 1H), 3.88-3.69 (m, 6H), 3.15-3.12 (m, 2H), 2.49 (s, 3H), 2.40 (s, 3H).

### <Example 30> Preparation of methyl 3-(6,7-dimethyl-3-oxo-4-((2S,3S,4R)-2,3,4,5-tetrahydroxypentyl)-3,4-dihydroquinoxaline-2-carboxamido)-4-methylbenzoate

The compound of Example 30 as a yellow solid was obtained in the same manner as in Example 1, except that aryl amine (III, R = a30) was used instead of aryl amine (III, R = a1) (24.6 mg, 56.3% yield).

ESI LC/MS: m/z calcd. for: C₂₅H₂₉N₃O₈ [M+H]⁺: 500.27; found 500.20 ¹H NMR (DMSO, 400MHz) *δ* 8.70 (d, *J =* 1.68 Hz 1H), 7.77 (s, 1H), 7.71 (q, *J =* 1.72 Hz 2H), 7.45 (d, *J =* 8.00 Hz, 1H), 5.03 (d, *J =* 4.56 Hz, 1H), 4.87 (q, *J =* 4.48 Hz, 1H), 4.74-4.70 (m, 2H), 4.49-4.47 (m, 1H), 4.31-4.28 (m, 1H), 4.18-4.17 (m, 1H), 3.63 (d, *J=* 3.92 Hz, 3H), 2.68-2.66 (m, 3H), 2.36 (s, 3H), 2.33-2.31(m, 3H).

### <Example 31> Preparation of N-(3-(1H-indol-3-yl)propyl)-6,7-dimethyl-3-oxo-4-((2S,3S,4R)-2,3,4,5-tetrahydroxypentyl)-3,4-dihydroquinoxaline-2-carboxamide

The compound of Example 31 as a yellow solid was obtained in the same manner as in Example 1, except that aryl amine (III, R = a31) was used instead of aryl amine (III, R = a1) (20 mg, .44.3% yield).

ESI LC/MS: m/z calcd. for: C₂₇H₃₂N₄O₆ [M+H]⁺: 509.29; found 509.24 ¹H NMR (MeOD, 400MHz) *δ* 7.65 (s, 1H), 7.61 (s, 1H), 7.53 (d, *J =* 8.00 Hz, 1H), 7.34 (d, *J =* 8.08 Hz, 1H), 7.16 (d, *J =* 2.24 Hz, 1H), 6.96-6,91 (m, 1H), 4.97 (d, *J =* 4.68 Hz, 1H), 4.85-4.82 (m, 1H), 4.67-4.58 (m, 2H), 4.47 (t, *J =* 5.54 Hz, 1H), 4.24-4.21 (m, 1H), 4.13-4.12 (m, 1H), 3.64-3.61 (m, 3H), 3.60-3.59 (m, 1H) 2.79 (t, *J =* 7.4 Hz, 1H), 2.68 (d, *J =* 7.24 Hz, 2H), 1.92-1.88 (m, 2H).

### <Example 32> Preparation of N-(1H-indazol-5-yl)-6,7-dimethyl-3-oxo-4-((2S,3S,4R)-2,3,4,5-tetrahydroxypentyl)-3,4-dihydroquinoxaline-2-carboxamide

The compound of Example 32 as a yellow solid was obtained in the same manner as in Example 1, except that aryl amine (III, R = a32) was used instead of aryl amine (III, R = a1) (2.8 mg, 6.10% yield).

ESI LC/MS: m/z calcd. for: C₂₃H₂₅N₅O₆ [M+H]⁺: 468; found 468.18 ¹H NMR (DMSO, 400MHz) *δ* 8.30 (s, 1H), 8.09 (s, 1H), 7.76-7.66 (m, 2H), 7.56-7.50 (m, 2H), 4.69-4.63 (m, 2H), 4.29-4.27 (m, 1H), 4.17-4.15 (m, 1H), 3.65-3.62 (m, 4H), 2.89 (s, 2H), 2.73-2.66 (m, 6H), 2.41 (s, 5H), 2.35 (s, 4H), 2.34-2.31 (m, 6H). (Mixture form : purity: 80%)

### <Example 33> Preparation of methyl 3-(6,7-dimethyl-3-oxo-4-((2S,3S,4R)-2,3,4,5-tetrahydroxypentyl)-3,4-dihydroquinoxaline-2-carboxamido)cyclopentane-1-carboxylate

The compound of Example 33 as a yellow solid was obtained in the same manner as in Example 1, except that aryl amine (III, R = a33) was used instead of aryl amine (III, R = a1) (25 mg, 46% yield).

ESI LC/MS: m/z calcd. for: C₂₃H₃₁N₃O₈ [M+H]⁺: 478.22; found 478.21 ¹H NMR (DMSO, 400MHz) *δ* 7.64 (s, 1H), 7.60 (s, 1H), 7.79 (s, 1H), 4.63-4.57 (m, 1H), 4.28-4.20 (m, 2H), 4.12-4.10 (m, 1H), 4.65-4.60 (m, 1H), 3.62 (s, 7H), 2.89-2.87 (m, 1H), 2.39 (s, 3H), 2.33-2.27 (m, 5H), 1.98-1.95 (m, 1H), 1.90-1.86 (m, 2H), 1.71-1.68 (m, 1H), 1.61-1.58 (m, 1H).

### <Example 34> Preparation of N-((1H-benzo[d]imidazol-5-yl)methyl)-6,7-dimethyl-3-oxo-4-((2S,3S,4R)-2,3,4,5-tetrahydroxypentyl)-3,4-dihydroquinoxaline-2-carboxamide

The compound of Example 34 as a yellow solid was obtained in the same manner as in Example 1, except that aryl amine (III, R = a34) was used instead of aryl amine (III, R = a1) (6 mg, 14.28% yield).

ESI LC/MS: m/z calcd. for: C₂₄H₂₇N₅O₆ [M+H]⁺: 482.24; found 482.20 ¹H NMR (DMSO, 400MHz) *δ* 7.79-7.76 (m, 2H), 7.64 (d, *J=* 10.1 Hz, 2H), 7.53-7.51 (m, 1H), 4.69-4.60 (m, 3H), 4.25-4.21 (m, 1H), 4.14 (d, *J =* 10.2 Hz, 1H), 3.63-3.60 (m, 3H), 2.89 (s, 1H), 2.39 (s, 3H), 2.33 (s, 3H).

### <Example 35> Preparation of N-(4-fluoro-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide

The compound of Example 35 as a yellow solid was obtained in the same manner as in Example 1, except that aryl amine (III, R = a35) was used instead of aryl amine (III, R = a1) (14 mg, 10.01% yield).

ESI LC/MS: m/z calcd. for: C₂₃H₂₄FN₅O₆ [M+H]⁺: 486.12; found 486.17 ¹H NMR (MeOD, 400MHz) *δ* 7.87 (s, 1H), 7.78 (s, 1H), 7.38 (d, *J =* 8.36 Hz, 1H), 7.22-7.17 (m, 1H), 7.00-6.95 (m, 1H), 4.63 -4.59 (m, 2H), 4.39-4.33 (m, 1H), 3.88-3.86 (m, 3H), 3.75-3.72 (m, 1H), 3.84-3.81 (m, 2H), 3.62 (t, *J =* 6.16 Hz, 3H), 2.51 (s, 3H), 2.43 (s, 3H).

### <Example 36> Preparation of N-(4-bromo-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide

The compound of Example 36 as a yellow solid was obtained in the same manner as in Example 1, except that aryl amine (III, R = a36) was used instead of aryl amine (III, R = a1) (10 mg, 11.68% yield).

ESI LC/MS: m/z calcd. for: C₂₃H₂₄BrN₅O₆ [M+H]⁺: 548.10; found 548.09 ¹H NMR (MeOD, 400MHz) *δ* 7.81 (s, 1H), 7.77 (s, 1H), 7.79 (s, 1H), 7.66 (d, *J =* 8.16 Hz, 1H), 7.58 (d, *J =* 7.88 Hz, 1H), 7.34-7.32 (m, 1H), 4.43 (d, *J =* 9.28 Hz, 1H), 3.90-3.86 (m, 3H), 3.77-3.74 (m, 1H), 3.50-3.48 (m, 1H), 3.15 (t, *J =* 1.68 Hz, 1H), 2.47 (s, 3H), 2.38 (s, 3H).

### Preparation of N-(2-(5-chloro-1H-indol-3-yl)ethyl)-6,7-dimethyl-3-oxo-4-((2S,3S,4R)-2,3,4,5-tetrahydroxypentyl)-3,4-dihydroquinoxaline-2-carboxamide

The compound of Example 37 as a yellow solid was obtained in the same manner as in Example 1, except that aryl amine (III, R = a37) was used instead of aryl amine (III, R = a1) (50 mg, 63.3% yield).

ESI LC/MS: m/z calcd. for: C₂₆H₂₉ClN₄O₆ [M+H]⁺: 529.17; found 529.18 ¹H NMR (DMSO, 400MHz) *δ* 7.66 (s, 2H), 7.61 (s, 1H), 7.37-7.33 (m, 2H), 7.72-7.64 (m, 3H), 7.08 (d, *J =* 1.88 Hz, 1H), 4.64-4.53 (m, 1H), 3.64-3.54 (m, 4H), 2.95-2.92 (m, 2H), 2.75 (s, 3H), 2.67 (d, *J =* 1.92 Hz, 1H), 2.39 (s, 3H), 2.33 (s, 3H).

### <Example 38> Preparation of N-(2-(4-fluoro-1H-benzo[d]imidazol-2-yl)ethyl)-6,7-dimethyl-3-oxo-4-((2S,3S,4R)-2,3,4,5-tetrahydroxypentyl)-3,4-dihydroquinoxaline-2-carboxamide

The compound of Example 38 as a yellow solid was obtained in the same manner as in Example 1, except that aryl amine (III, R = a38) was used instead of aryl amine (III, R = a1) (35 mg, 29.2% yield).

ESI LC/MS: m/z calcd. for: C₂₅H₂₈FN₅O₆ [M+H]⁺: 514.08; found 514.21 ¹H NMR (MeOD, 400MHz) *δ* 7.79-7.76 (m, 1H), 7.61 (s, 1H), 7.55-7.53 (d, 2H), 7.40-7.35 (m, 1H), 4.73-4.68 (m, 1H), 4.33-4.30 (m, 1H), 4.24-4.22 (m, 1H), 4.02 (d, *J =* 3.8 Hz, 2H), 3.86-3.78 (m, 3H), 3.72-3.69 (m, 1H), 3.60-3.57 (m, 2H), 2.48 (s, 3H), 2.34 (s, 3H).

### <Example 39> Preparation of N-(4-hydroxy-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide

The compound of Example 39 as a yellow solid was obtained in the same manner as in Example 1, except that aryl amine (III, R=a39) was used instead of aryl amine (III, R=a1) (2 mg, 1.26% yield).

ESI LC/MS: m/z calcd. for: C₂₃H₂₅N₅O₇[M+H]⁺: 483.99; found 484.18 ¹H NMR (MeOD, 400MHz) *δ* 7.92 (s, 1H), 7.84 (s, 1H), 7.31 (t, *J =* 7.92 Hz, 1H), 7.21 (d, *J =* 8.04 Hz, 1H), 6.91 (d, *J =* 8.04 Hz, 1H), 4.13 (q, *J =* 7.20 Hz 1H), 3.86 (s, 3H), 3.18 (s, 1H), 3.01 (s, 2H), 2.55 (s, 3H), 2.46 (s, 3H), 2.33 (s, 2H).

### <Example 40> Preparation of 3-(2-(6,7-dimethyl-3-oxo-4-((2S,3S,4R)-2,3,4,5-tetrahydroxypentyl)-3,4-dihydroquinoxaline-2-carboxamido)ethyl)-1H-indol-5-yl pivalate

The compound of Example 40 as a yellow solid was obtained in the same manner as in Example 1, except that aryl amine (III, R=a40) was used instead of aryl amine (III, R=a1) (6 mg, 34.7% yield).

ESILC/MS: m/z calcd. for: C₃₁H₃₈N₄O₈[M+H]⁺: 595.17; found 595.27 ¹H NMR (MeOD, 400MHz) *δ* 7.82 (s, 1H), 7.70 (s, 1H), 7.35-7.37 (m, 1H), 7.25-7.21 (m, 2H), 6.75 (d, *J* = 8.24 Hz, 1H), 4.57 (d, *J* = 7.32 Hz, 1H), 4.46-4.42 (m, 1H), 4.13 (d, *J=* 3.64 Hz, 1H), 3.82-3.76 (m, 6H), 3.12-3.08 (m, 2H), 2.48 (s, 3H), 2.41 (s, 3H), 1.29 (s, 9H).

Compounds of Examples 41 to 45 were prepared by the method of the following Reaction Scheme B.

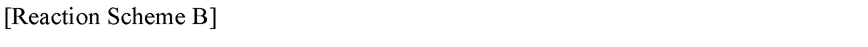

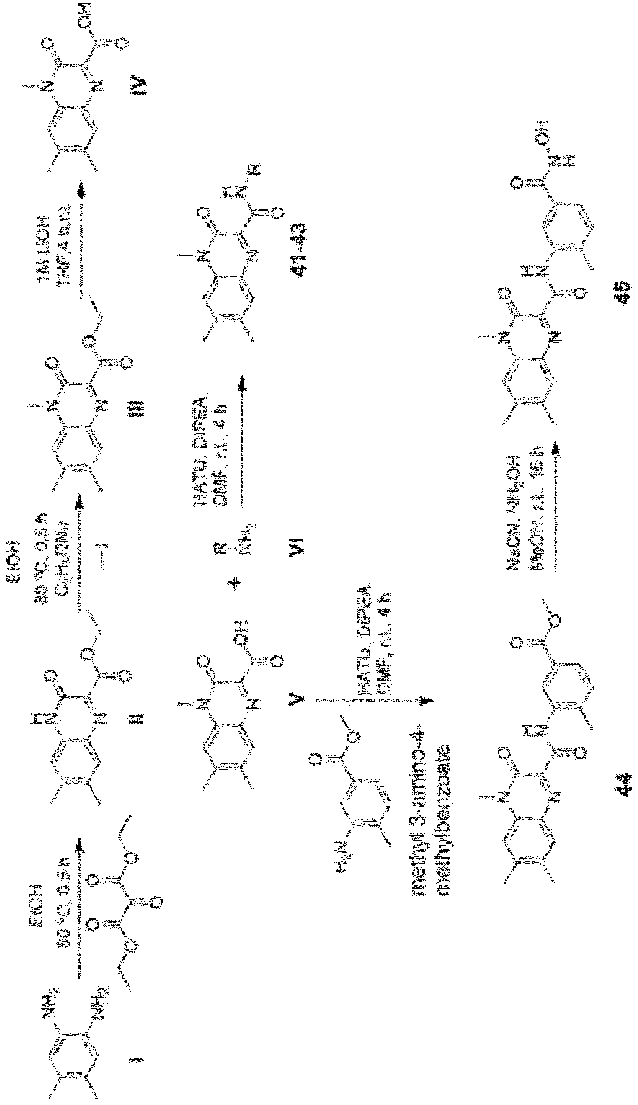

In the Reaction Scheme B, -R of Compound VI is b1 to b3 as follows.

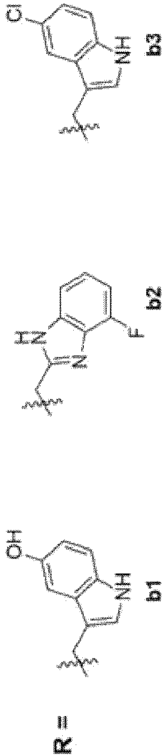

Hereinafter, specific preparation methods of Examples 41 to 45 are described.

### <Example 41> Preparation of N-(2-(5-hydroxy-1H-indol-3-yl)ethyl)-4,6,7-trimethyl-3-oxo-3,4-dihydroquinoxaline-2-carboxamide

In the Reaction Scheme B, Compound VI (R=b1) was used to obtain the compound of Example 41 as a yellow solid (22 mg, 24% yield).

ESILC/MS: m/z calcd. for: C₂₂H₂₂N₄O₃[M+H]⁺: 391.85; found 391.17 ¹H NMR (DMSO, 400MHz) *δ* 7.67 (s, 1H), 7.45 (s, 1H), 7.14 (q, *J =* 4.72 Hz, 2H), 6.88 (d, *J =* 2.0 Hz, 1H), 6.61 (q, *J =* 2.16 Hz, 1H), 3.55(s, 3H), 3.54-3.52 (m, 2H), 2.88-3.84 (m, 2H), 2.42 (s, 3H), 2.33 (s, 3H).

### <Example 42> Preparation of N-(2-(4-fluoro-1H-benzo[d]imidazol-2-yl)ethyl)-4,6,7-trimethyl-3-oxo-3,4-dihydroquinoxaline-2-carboxamide

In the Reaction Scheme B, Compound VI (R=b2) was used to obtain the compound of Example 42 as a yellow solid (28 mg, 38% yield).

ESI LC/MS: m/z calcd. for: C₂₁H₂₀FN₅O₂[M+H]⁺: 394.05; found 394.16 ¹H NMR (DMSO, 400MHz) *δ* 7.74-7.71 (m, 1H), 7.63 (s, 1H), 7.79 (s, 1H), 7.60 (d, *J=* 9.08 Hz, 1H), 7.47 (s, 1H), 7.30 (t, *J =* 9.00 Hz, 1H), 3.82-3.80 (m, 2H), 3.64 (s, 4H), 2.67 (d, *J =* 1.8 Hz, 1H), 2.42 (s, 3H), 2.33 (s, 3H).

### <Example 43> Preparation of N-(2-(5-chloro-1H-indol-3-yl)ethyl)-4,6,7-trimethyl-3-oxo-3,4-dihydroquinoxaline-2-carboxamide

In the Reaction Scheme B, Compound VI (R=b3) was used to obtain the compound of Example 43 as a yellow solid (22 mg, 10.5% yield).

ESI LC/MS: m/z calcd. for: C₂₂H₂₁ClN₄O₂[M+H]⁺: 409.02; found 409.14 ¹H NMR (DMSO, 400MHz) *δ* 7.67 (s, 1H), 7.65 (d, *J =* 1.84Hz, 1H), 7.45 (s, 1H), 7.37 (d, *J =* 8.6 Hz, 1H), 7.32 (s, 1H), 7.08-7.05 (m, 1H), 3.65 (s, 1H), 3.59 (t, *J =* 7.16 Hz, 2H), 2.97 (t, *J* = 7.16 Hz, 2H), 2.43 (s, 3H), 2.34 (s, 3H).

### <Example 44> Preparation of methyl 4-methyl-3-(4,6,7-trimethyl-3-oxo-3,4-dihydroquinoxaline-2-carboxamido)benzoate

In the Reaction Scheme B, methyl 3-amino-4-methylbenzoate was added to Compound V to obtain the compound of Example 44 as a yellow solid (102 mg, 62.4% yield).

ESI LC/MS: m/z calcd. for: C₂₁H₂₂N₃O₄[M+H]⁺: 380.16; found 380.03.

### <Example 45> Preparation of N-(5-(hydroxycarbamoyl)-2-methylphenyl)-4,6,7-trimethyl-3-oxo-3,4-dihydroquinoxaline-2-carboxamide

After the compound of Example 44 was mixed with THF, the mixture was added to 0.5 equivalents of sodium cyanide and a methanol solution. Then, 20 equivalents of a 50% hydroxylamine aqueous solution were added, and the resulting mixture was stirred at room temperature for 16 hours. After concentrating the mixed solution in vacuo, the resulting residue was filtered through a Microfilter and purified using preparative HLPC (solvent system: acetonitrile, water) to obtain the compound of Example 45 as a yellow solid (2.9 mg, 13.84% yield).

ESILC/MS: m/z calcd. for: C₂₀H₂₀N₄O₄[M+H]⁺: 381.05; found 381.15 ¹H NMR (MeOD, 400MHz) *δ* 8.40 (s, 1H), 7.80 (d, *J =* 8.16 Hz, 1H), 7.55 (s, 1H), 7.47 (d, 3H), 7.36 (d, *J =* 8.10 Hz, 1H), 3.73 (s, 3H), 2.67 (s, 1H), 2.33 (s, 6H), 2.29 (s, 1H), 1.24 (s, 2H).

Compounds of Examples 46 to 52 were prepared by the method of the following Reaction Scheme C.

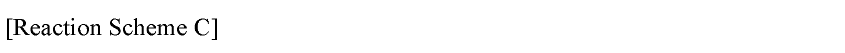

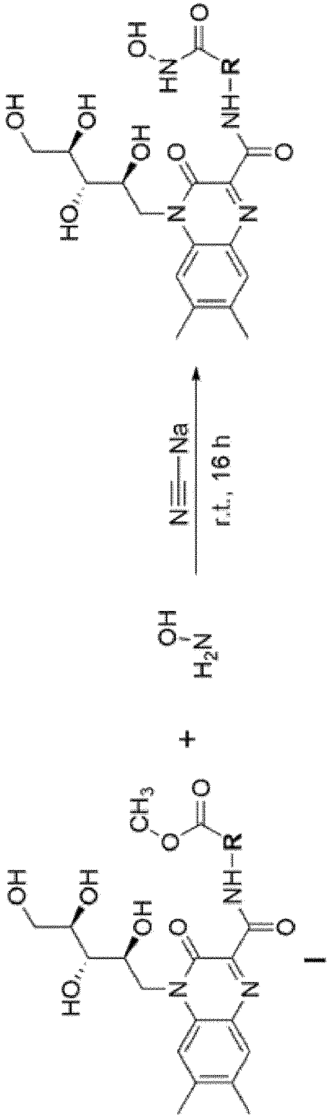

In the Reaction Scheme C, -R of Compound I is c1 to c7 as follows.

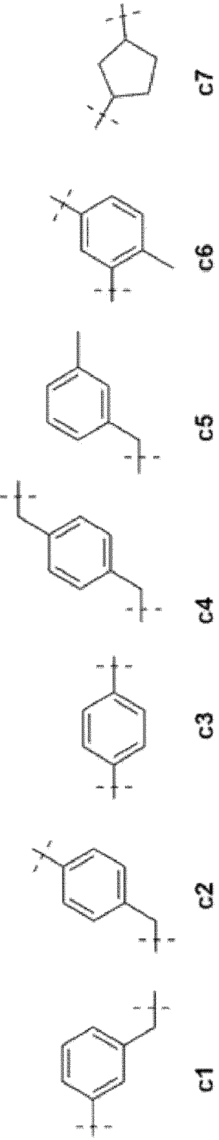

Hereinafter, specific preparation methods of Examples 46 to 52 are described.

### <Example 46> Preparation of N-(3-(2-(hydroxyamino)-2-oxoethyl)phenyl)-6,7-dimethyl-3-oxo-4-((2S,3S,4R)-2,3,4,5-tetrahydroxypentyl)-3,4-dihydroquinoxaline-2-carboxamide

In the Reaction Scheme C, Compound I (R=c1) was used to obtain the compound of Example 46 as a yellow solid (7 mg, 46.6% yield).

ESI LC/MS: m/z calcd. for: C₂₄H₂₈N₄O₈ [M+H]⁺: 501.50; found 501.20. ¹H NMR (CD₃OD, 400MHz) δ 11.13 (s, 1H), 7.70 (s, 1H), 7.68-7.64 (m, 2H), 7.46 (s, 1H), 7.22 (t, *J* = 7.8 Hz, 1H), 6.99 (d, *J* = 7.64 Hz, 1H), 4.67 (q, *J =* 10.24 Hz, 1H), 4.28 (d, *J =* 11.72 Hz, 1H), 4.15 (d, *J =* 9.56 Hz, 1H), 3.65-3.61 (m, 3H), 3.47-3.45 (s, 1H), 3.10 (s, 2H), 2.40 (s, 3H), 2.33 (s, 3H).

### <Example 47> Preparation of N-(4-(hydroxycarbamoyl)benzyl)-6,7-dimethyl-3-oxo-4-((2S,3S,4R)-2,3,4,5-tetrahydroxypentyl)-3,4-dihydroquinoxaline-2-carboxamide

In the Reaction Scheme C, Compound I (R=c2) was used to obtain the compound of Example 47 as a yellow solid (3.1 mg, 30.9% yield).

ESI LC/MS: m/z calcd. for: C₂₄H₂₈N₄O₈ [M+H]⁺: 501.50; found 501.23. ¹H NMR (CD₃OD, 400MHz) δ 7.82-7.80 (m, 2H), 7.77-7.75 (m, 1H), 7.71-7.70 (m, 1H), 7.53 (d, *J =* 8.2 Hz, 1H), 7.45 (d, *J =* 8.4 Hz, 1H), 4.75 (s, 1H), 4.72 (s, 2H), 4.49 (dd, *J =* 13.96, 2.6 Hz, 1H), 4.31-4.28 (m, 1H), 3.85-3.78 (m, 3H), 3.72-3.67 (m, 1H), 2.50 (s, 3H), 2.41 (s, 3H).

### <Example 48> Preparation of N-(4-(hydroxycarbamoyl)phenyl)-6,7-dimethyl-3-oxo-4-((2S,3S,4R)-2,3,4,5-tetrahydroxypentyl)-3,4-dihydroquinoxaline-2-carboxamide

In the Reaction Scheme C, Compound I (R=c3) was used to obtain the compound of Example 48 as a yellow solid (2.4 mg, 15.9% yield).

ESI LC/MS: m/z calcd. for: C₂₃H₂₆N₄O₈ [M+H]⁺: 487.47; found 487.20. ¹H NMR (CD₃OD, 400MHz) δ 7.91-7.88 (m, 2H), 7.83-7.79 (m, 3H), 7.72 (s, 1H), 4.53 (dd, *J =* 14, 2.6 Hz, 2H), 4.35-4.31 (m, 1H), 3.86-3.79 (m, 3H), 3.72-3.68 (m, 1H), 2.49 (s, 3H), 2.41 (s, 3H).

### <Example 49> Preparation of N-(4-(2-(hydroxyamino)-2-oxoethyl)benzyl)-6,7-dimethyl-3-oxo-4-((2S,3S,4R)-2,3,4,5-tetrahydroxypentyl)-3,4-dihydroquinoxaline-2-carboxamide

In the Reaction Scheme C, Compound I (R=c4) was used to obtain the compound of Example 49 as a yellow solid (1.2 mg, 13.2% yield).

ESI LC/MS: m/z calcd. for: C₂₅H₃₀N₄O₈ [M+H]⁺: 515.52; found 515.24. ¹H NMR (CD₃OD, 400MHz) δ 7.81 (s, 1H), 7.70 (s, 1H), 7.39 (d, *J =* 8.24 Hz, 2H), 7.31 (d, *J =* 8.16 Hz, 2H), 4.77 (q, *J =* 9.96 Hz, 1H), 4.67 (s, 2H), 4.47 (dd, *J =* 13.64, 2.12 Hz, 1H), 4.30-4.26 (m, 1H), 3.85-3.76 (m, 3H), 3.71-3.67 (m, 1H), 3.42 (s, 2H), 2.50 (s, 3H), 2.41 (s, 3H).

### <Example 50> Preparation of N-(3-(hydroxycarbamoyl)benzyl)-6,7-dimethyl-3-oxo-4-((2S,3S,4R)-2,3,4,5-tetrahydroxypentyl)-3,4-dihydroquinoxaline-2-carboxamide

In the Reaction Scheme C, Compound I (R=c5) was used to obtain the compound of Example 50 as a yellow solid (1.5 mg, 14.9% yield).

ESI LC/MS: m/z calcd. for: C₂₄H₂₈N₄O₈[M+H]⁺: 501.50; found 501.17. ¹H NMR (CD₃OD, 400MHz) *δ* 7.84-7.79 (m, 2H), 7.73-7.66 (m, 2H), 7.64-7.59 (m, 1H), 7.46 (t, J= 7.68 Hz, 1H), 4.80-4.71 (m, 3H), 4.58 (s, 1H), 4.47 (dd, J= 13.92, 2.48 Hz, 1H), 4.29-4.26 (m, 1H), 3.83-3.75 (m, 3H), 3.69-3.65 (m, 1H), 2.48 (s, 3H), 2.39 (s, 3H).

### <Example 51> Preparation of N-(5-(hydroxycarbamoyl)-2-methylphenyl)-6,7-dimethyl-3-oxo-4-((2S,3S,4R)-2,3,4,5-tetrahydroxypentyl)-3,4-dihydroquinoxaline-2-carboxamide

In the Reaction Scheme C, Compound I (R=c6) was used to obtain the compound of Example 51 as a yellow solid (3.2 mg, 21.9% yield).

ESI LC/MS: m/z calcd. for: C₂₄H₂₈N₄O₈ [M+H]⁺: 501.50; found 501.21 ¹H NMR (CD₃OD, 400MHz) *δ* 8.62 (s, 1H), 7.83 (s, 1H), 7.71 (s, 1H), 7.49 (dd, *J =* 7.84, 1.72 Hz, 1H), 7.36 (d, *J =* 7.96 Hz, 1H), 4.58 (s, 1H), 4.55 (dd, *J =* 14.04, 2.52 Hz, 1H), 4.38-4.34 (m, 1H), 3.86-3.81 (m, 3H), 3.73-3.69 (m, 1H), 2.46 (s, 3H), 2.45 (s, 3H), 2.38 (s, 3H).

### <Example 52> Preparation of N-(3-(hydroxycarbamoyl)cyclopentyl)-6,7-dimethyl-3-oxo-4-((2S,3S,4R)-2,3,4,5-tetrahydroxypentyl)-3,4-dihydroquinoxaline-2-carboxamide

In the Reaction Scheme C, Compound I (R=c7) was used to obtain the compound of Example 52 as a yellow solid (1.8 mg, 8.72% yield).

ESI LC/MS: m/z calcd. for: C₂₂H₃₀N₄O₈ [M+H]⁺: 479.22; found 479.21 ¹H NMR (MeOD, 400MHz) δ 7.80 (s ,1H), 7.70 (s, 1H), 7.79 (s, 1H), 4.53-4.48 (m, 2H), 4.31-4.28 (m, 1H), 3.87-3.73 (m, 3H), 3.71-3.68 (m, 1H), 3.32 (s, 1H), 2.74-2.70 (m, 1H), 2.49 (s, 3H), 2.41-2.32 (m, 4H), 2.16-2.12 (m, 1H), 2.02-1.96 (m, 2H), 1.90-1.84 (m, 2H).

The compounds of Examples 1 to 52 are summarized and presented in Table 1 below.

**TABLE 1**

| No. | Chemical Formula | No. | Chemical Formula |
|---|---|---|---|
| 1 | 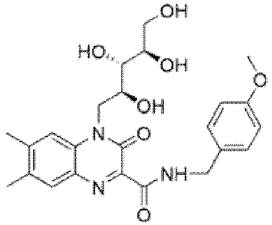 | 2 | 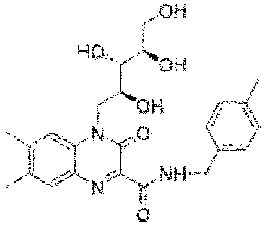 |
| 3 | 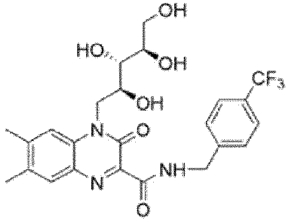 | 4 | 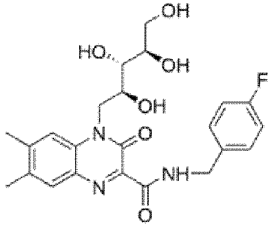 |
| 5 | 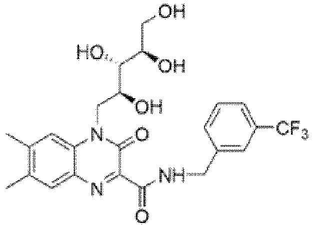 | 6 | 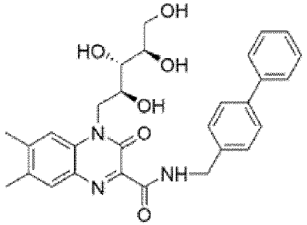 |
| 7 | 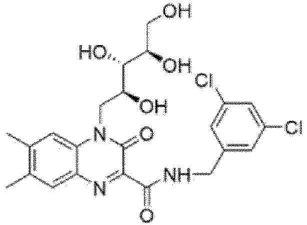 | 8 | 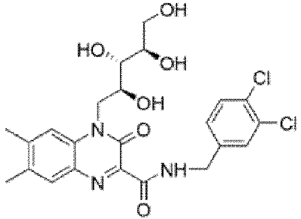 |
| 9 | 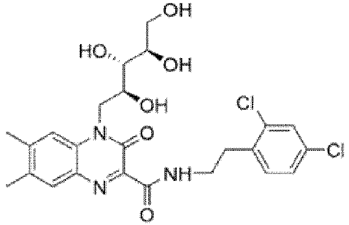 | 10 | 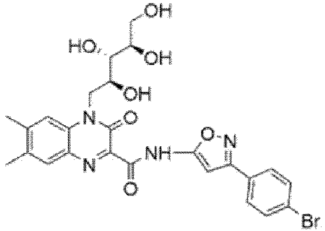 |
| 11 | 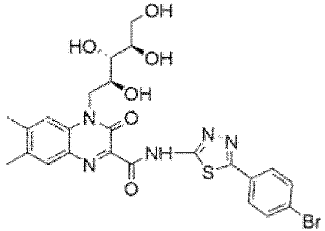 | 12 | 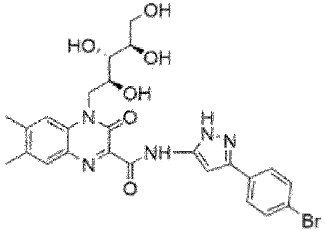 |
| 13 | 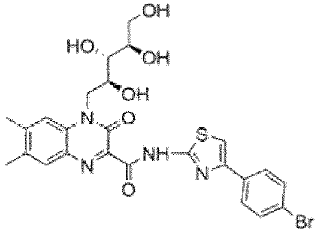 | 14 | 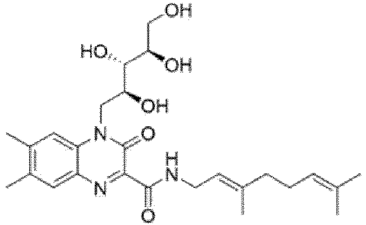 |
| 15 | 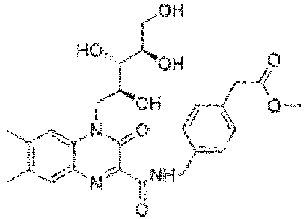 | 16 | 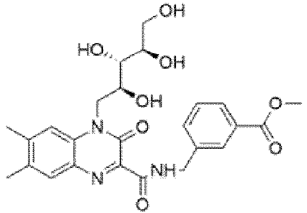 |
| 17 | 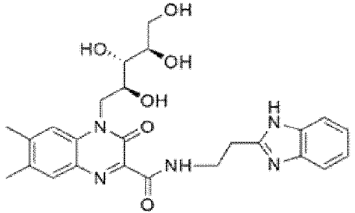 | 18 | 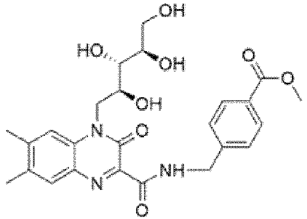 |
| 19 | 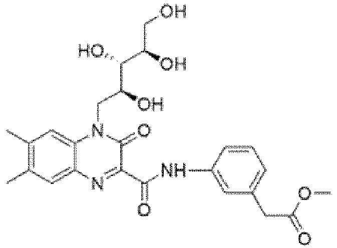 | 20 | 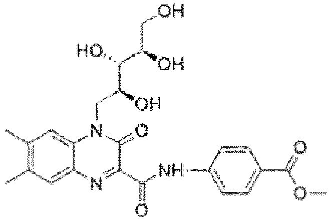 |
| 21 | 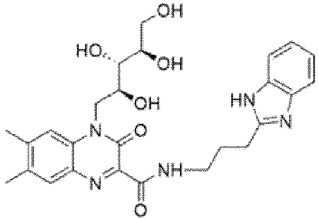 | 22 | 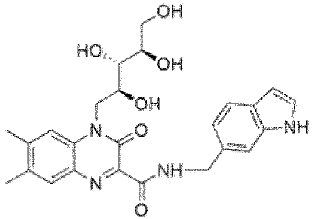 |
| 23 | 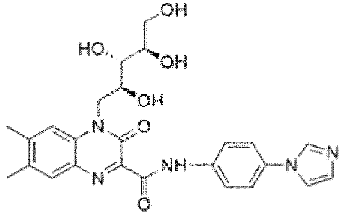 | 24 | 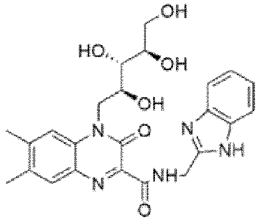 |
| 25 | 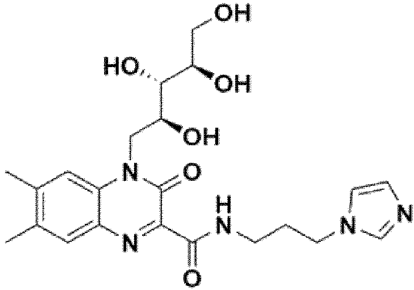 | 26 | 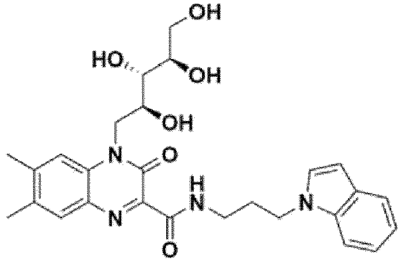 |
| 27 | 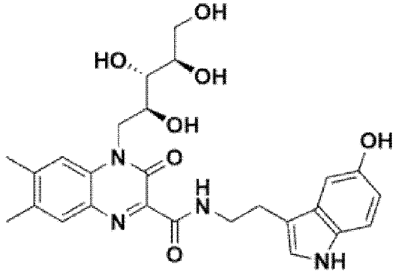 | 28 | 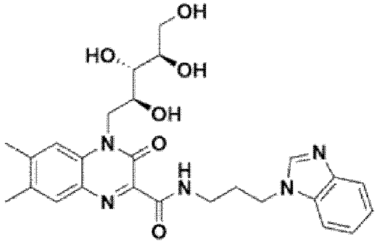 |
| 29 | 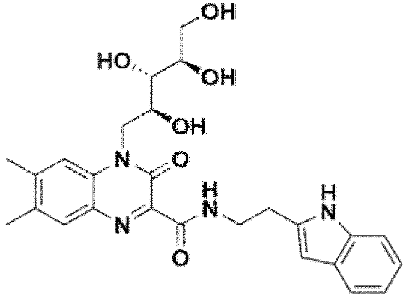 | 30 | 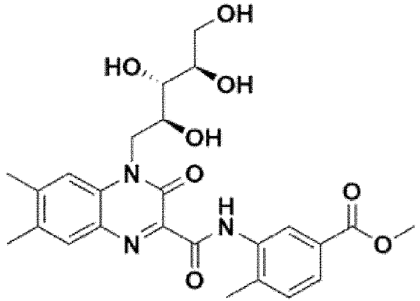 |
| 31 | 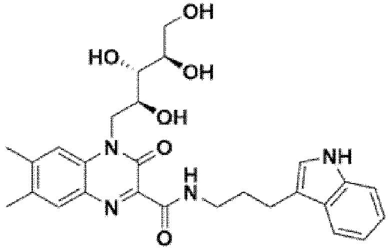 | 32 | 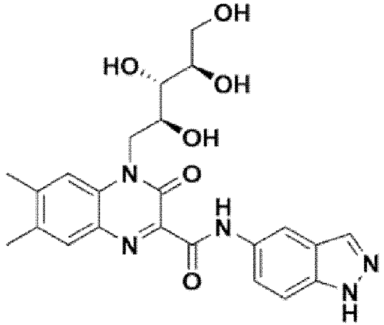 |
| 33 | 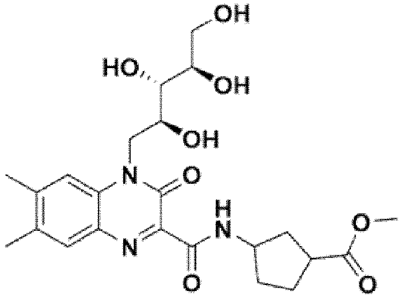 | 34 | 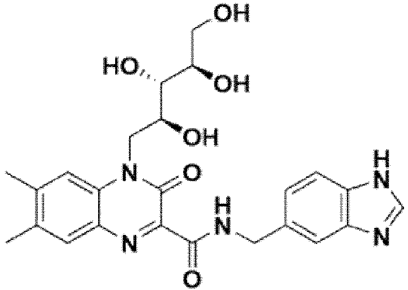 |
| 35 | 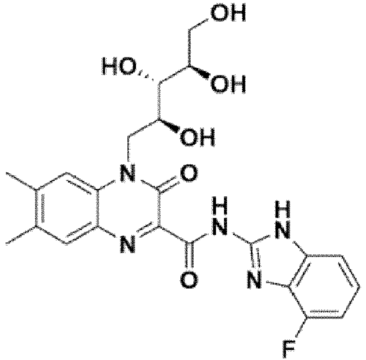 | 36 | 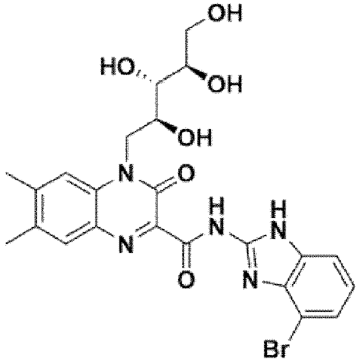 |
| 37 | 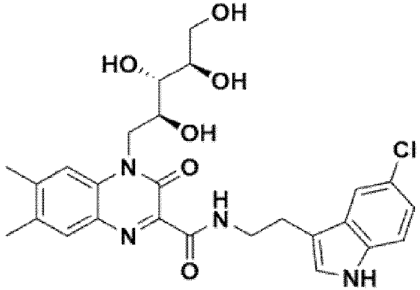 | 38 | 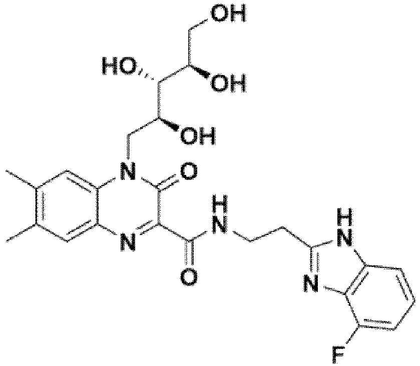 |
| 39 | 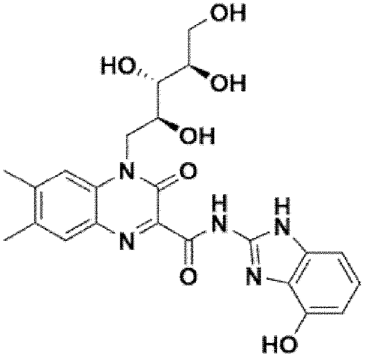 | 40 | 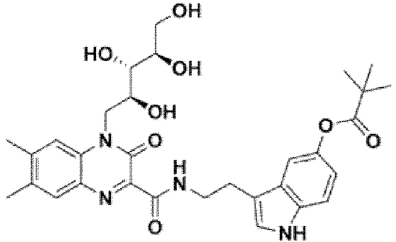 |
| 41 | 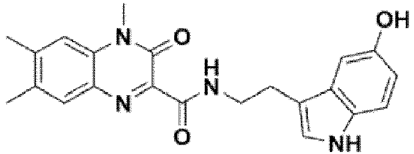 | 42 | 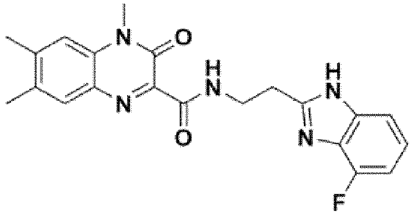 |
| 43 | 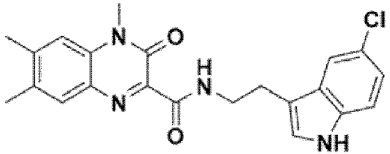 | 44 | 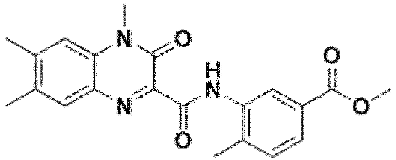 |
| 45 | 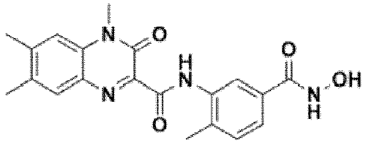 | 46 | 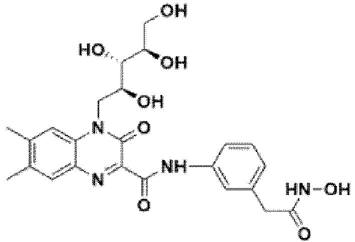 |
| 47 | 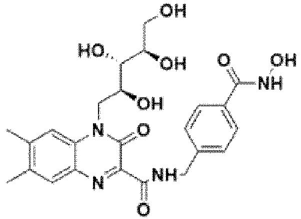 | 48 | 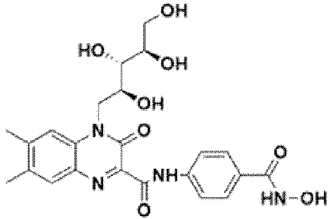 |
| 49 | 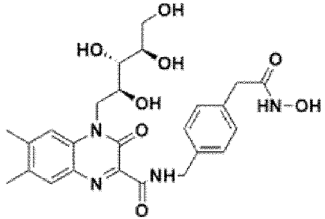 | 50 | 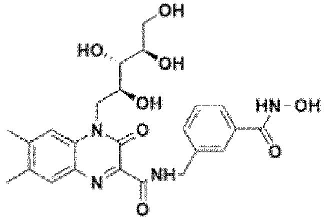 |
| 51 | 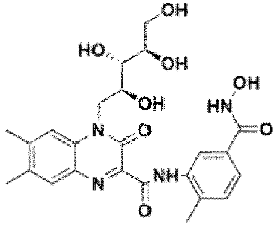 | 52 | 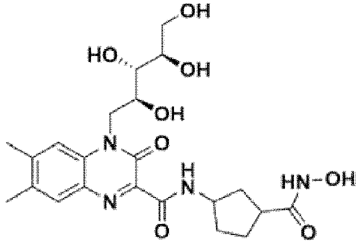 |

### <Comparative Example>

TSA (Trichostatin A), SAHA (suberoylanilide hydroxamic acid), and PCI-34051 (N-hydroxy-1-(4-methoxybenzyl)-1H-indole-6-carboxamide), which are known to inhibit HDAC8, were prepared as Comparative Examples.
TSA: 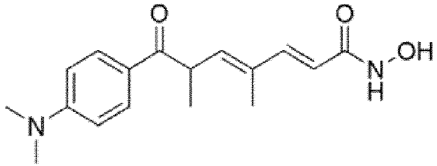
SARA: 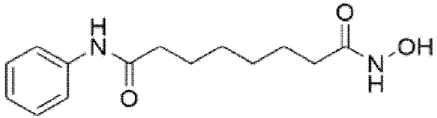
PCI-34051:

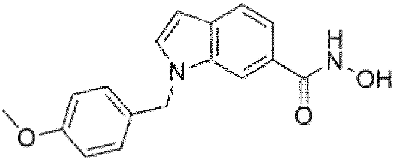

### Cell Culture

PC-3, a human-derived prostate cancer cell line, was cultured with RPMI1640 (10% fetal bovine serum, 1% Penicillin Streptomycin) culture medium at 37°C, 5% CO₂ condition. All cells in the experiment were subcultured when they reached a confluency of approximately 80% to 90%, and only cells that had not passed 20 passages were used.

### Statistical Analysis

All data are expressed as means ± standard deviation (SD) from at least three independent tests, and statistical significance of differences was determined by unpaired Student's test using GrahPad Prism 5. A P value less than 0.05 was considered statistically significant.

### <Experimental Example 1> HDAC8 inhibitory activity analysis (in vitro) - RFU analysis

To confirm the HDAC enzyme activity of the compound of the Example, the HDAC Fluorogenic assay kit from BPS bioscience was used for measurement, and the HDAC substrate 2A was diluted in buffer to a concentration of 20 µM and used for analysis. The compound of the Example was diluted to 1 mM (1% DMSO concentration) using DMSO to obtain a final concentration of 10 µM, and prepared by diluting 1/10 with assay buffer. In a 96-well black round-bottom plate, 5 µL of the diluted compound of the Example, 5 µL of the diluted HDAC substrate 2A, 5 µL of BSA (1 mg/mL), 5 µL of the HDAC8 enzyme diluted to an appropriate concentration, and 30 µL of assay buffer were added, and the enzyme reaction was performed at 37°C for 30 minutes. Blank does not contain HDAC8 enzyme, positive control is 100% value of HDAC8 enzyme activity with 1% DMSO, inhibitor control used TSA, SAHA, and PCI-34051 compounds. Afterwards, 50 µL of 2X HDAC developer was added to each well. After reacting for 15 minutes at room temperature, the fluorescence values at excitation wavelengths of 350-380 nm and emission wavelengths of 440-460 nm were measured using a microplate reader (Synergy Neo). The result values were calculated by averaging the blank values, subtracting the average blank value from all values, and setting the positive control value to 100.

The results are shown in Table 2 below, through which it was confirmed that the 3,4-dihydroquinoxaline-2-carboxamide derivative compound of the Example exhibited inhibitory activity against HDAC8, and in particular, the compounds of Examples 10, 27, 34, 35, 48, 49, and 51 exhibited inhibitory activity similar to TSA or SAHA, which are known as existing HDAC8 inhibitors.

**TABLE 2**

| | RFU (%) | | RFU (%) |
|---|---|---|---|
| DMSO | 100 | TSA | 13.83 |
| SAHA | 38.57 | PCI-34051 | 1.86 |
| 4 | 85.71 | 7 | 54.79 |
| 8 | 85.37 | 9 | 66.28 |
| 10 | 16.61 | 11 | 63.24 |
| 12 | 58.97 | 24 | 42.03 |
| 27 | 7.62 | 33 | 69.48 |
| 34 | 34.05 | 35 | 8.23 |
| 47 | 53.58 | 48 | 10.48 |
| 49 | 35.15 | 50 | 51.23 |
| 51 | 5.81 | 52 | 41.96 |

### <Experimental Example 2> Analysis of selective inhibitory effect on HDAC8

To confirm the selective inhibitory effect of the compound of the Example on HDAC8, the inhibitory activity was measured using the HDAC Fluorogenic assay kit from BPS bioscience. For analysis, HDAC substrate 3 was diluted in buffer to a concentration of 200 µM and used in HDAC 1, 2, 3, 6, and 10 assays, HDAC substrate 2A was diluted in buffer to a concentration of 200 µM and used in HDAC 4, 7, 9, and 11 assays, HDAC substrate 2A was diluted in buffer to a concentration of 20 µM and used in HDAC 8 assays. For the HDAC 11 assay, a dedicated buffer included in the kit was used separately, and the same buffer was used for the remaining assays. The compound of the Example was diluted to 1 mM (1% DMSO concentration) using DMSO to obtain a final concentration of 10 µM, and prepared by diluting 1/10 with assay buffer. In a 96-well black round-bottom plate, 5 µL of the diluted compound of the Example, 5 µL of the diluted HDAC substrate, 5 µL of BSA (1 mg/ml), 5 µL of the HDAC enzyme diluted to the appropriate concentration, and 30 µL of assay buffer were added, and the enzyme reaction was performed at 37°C for 30 minutes. Blank does not contain HDAC enzyme, positive control uses 100% enzyme activity value with 1% DMSO, and inhibitor control used TSA compound. Afterwards, 50 µL of 2X HDAC developer was added to each well. After reacting for 15 minutes at room temperature, the fluorescence values at excitation wavelengths of 350-380 nm and emission wavelengths of 440-460 nm were measured using a microplate reader (Synergy Neo). The result values were calculated by averaging the blank values, subtracting the average blank value from all values, and setting the positive control value for each HDAC enzyme to 100.

The results are shown in Table 3 below. As a result of the experiment, TSA, known as an existing HDAC8 inhibitor, showed a very high inhibitory effect not only on HDAC8 but also on HDAC1, HDAC2, HDAC6, and HDAC10, and also showed a high inhibitory effect on HDAC3, HDAC4, HDAC7, and HDAC9, so a selective inhibitory effect on HDAC8 could not be obtained. On the other hand, the compound of Example 27 showed selective and excellent inhibitory activity only against HDAC8.

**TABLE 3**

| | DMSO (%) | TSA (%) | Example 27 (%) |
|---|---|---|---|
| HDAC1 | 100 | 1.22 | 76.23 |
| HDAC2 | 100 | 0.70 | 91.24 |
| HDAC3 | 100 | 21.38 | 73.14 |
| HDAC4 | 100 | 18.83 | 98.24 |
| HDAC6 | 100 | 1.11 | 43.66 |
| HDAC7 | 100 | 8.91 | 91.19 |
| HDAC8 | 100 | 11.05 | 14.05 |
| HDAC9 | 100 | 21.46 | 66.46 |
| HDAC10 | 100 | 1.21 | 87.15 |
| HDAC11 | 100 | 59.06 | 97.30 |

### <Experimental Example 3> Analysis of prostate cancer and liver cancer cells growth inhibitory activity

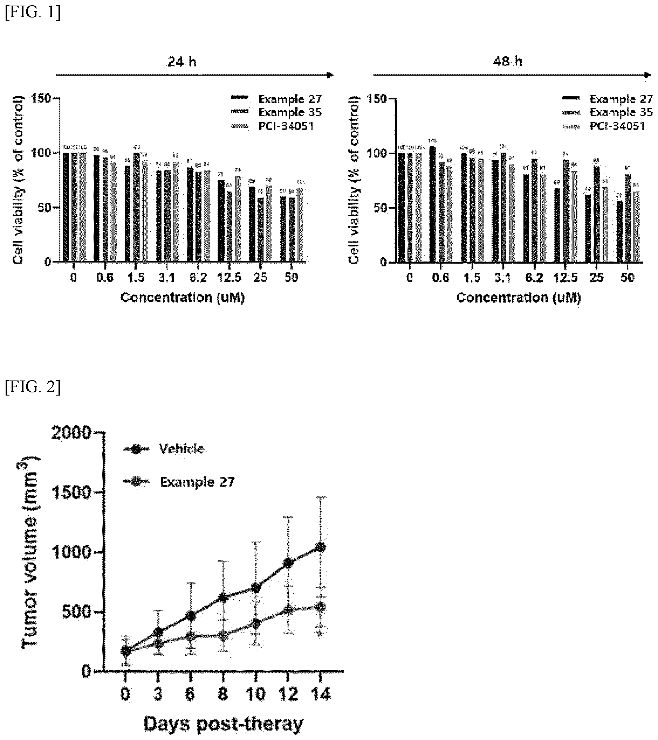

In order to analyze the growth inhibitory effect of the compounds of the Example on prostate cancer cells, PC-3 prostate cancer cells were treated with various concentrations of compounds of Example 27 and Example 35 and PCI-34051, and then cell viability was confirmed on day 1 and day 2 through a CCK-8 assay. Specifically, PC-3 cells were dispensed into a 96-well plate at 1 × 10⁴ cells/mL, and after 20 hours, PCI34051, Example 27, and Example 35 compounds were added at various concentrations, and cultured for 24 hours and 48 hours in a 37°C, 5% CO₂ incubator. After culturing, 10 µL CCK-8 reagent was added and the absorbance was measured after 1 hour. The absorbance was measured at 450 nm using a microplate reader [BMG Labtech (Offenburg, Germany)]. The results are shown in FIG. 1. It was confirmed that PCI-34051, known as an HDAC8 inhibitor, inhibits cancer cell viability in a concentration- and time-dependent manner, and the compound of Example 27 also effectively reduced the viability of prostate cancer cells in a concentration- and time-dependent manner, and showed a cancer cell viability inhibition rate superior to that of PCI-34051. The compound of Example 35 showed a concentration-dependent inhibition of cancer cell viability after 24 hours, although cell viability was restored after 48 hours.

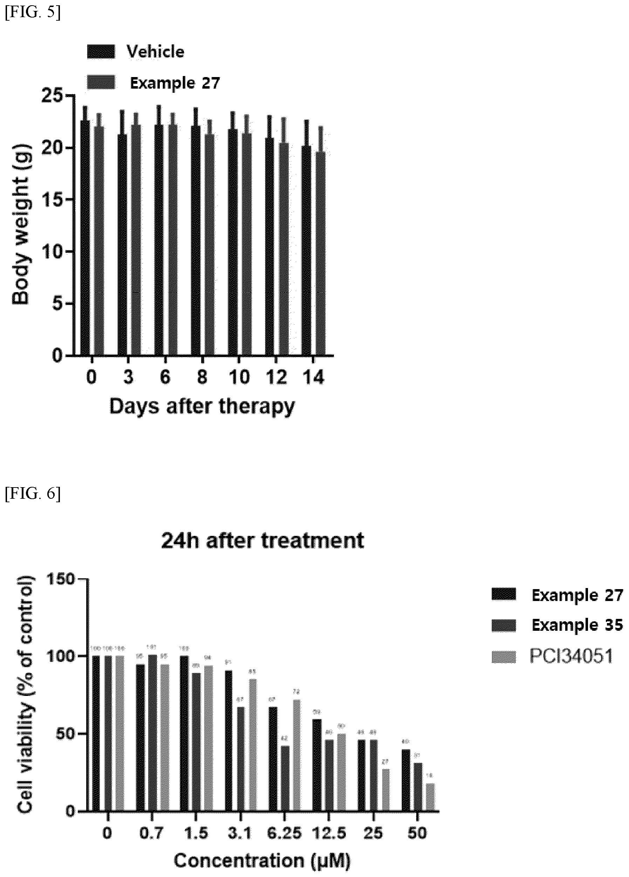

In addition, an analysis of liver cancer growth inhibitory activity was performed. Liver cancer cells Huh-7 cells were dispensed in a 96-well plate at 1 × 10⁴ cells/ml, and after 20 hours, PCI34051, Example 27, and Example 35 were added at various concentrations, and cultured for 24 and 48 hours at 37°C in a 5% CO₂ atmosphere. After culturing, 100 µl CellTiter-Glo Luminescent Cell Viability Assay reagent was added and the luminescence signal was measured after 0.5-1 hour. The luminescence signal was measured using IVIS [PerkinElmer, (Waltham, USA)]. After treating the liver cancer cell line Huh-7 with PCI-34051 (control), Examples 27 and 35 at various concentrations, the cell viability was confirmed using CellTiter-Glo Luminescent Cell Viability Assay on day 1, and as a result, as shown in FIG. 6, Examples 27 and 35 showed a concentration-dependent inhibition of cancer cell viability, as in the control.

### <Experimental Example 4> Analysis of anticancer effects on prostate cancer and liver cancer

A prostate cancer model was established by subcutaneously transplanting PC-3 cell lines into nude mice, and the mice were divided into two groups (group 1: vehicle administration group (10% DSMO, 30% PEG400, 60% DW), group 2: 50 mg/kg Example 27 compound administration group). The drug was administered orally once a day for 12 times. To evaluate the therapeutic efficacy, tumors were excised before drug administration and on days 3, 6, 8, 10, 12, and 14 after drug administration, and tumor volumes were measured. Tumor volume (mm³) was calculated as follows (tumor volume (mm³) = (A X B²)/2, where A = tumor long axis, B = tumor short axis), and the results are shown in FIG. 2. The vehicle administration group (group 1) showed significant tumor growth up to 14 days after administration, and the Example 27 compound administration group (group 2) showed a significantly reduced tumor volume growth rate compared to group 1, and statistical significance was confirmed (*p<0.05, compared with vehicle).

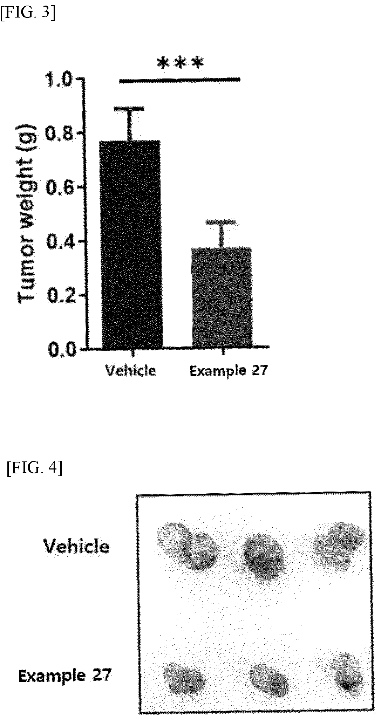

Meanwhile, on day 12 of drug administration, the tumor was removed and its weight was measured, the results are shown in FIG. 3, and a photograph of the removed tumor was taken and shown in FIG. 4. The vehicle administration group (group 1) had a significantly higher tumor weight than the Example 27 compound administration group (group 2), and statistical significance was also confirmed (***p<0.0005, compared with vehicle).

In addition, when the body weight of the mice was checked during the drug administration period, no rapid weight loss was confirmed (FIG. 5).

In addition, a liver cancer model was established by subcutaneously transplanting Huh-7 cell lines into nude mice. After establishing the liver cancer model, the mice were divided into two groups (group 1: vehicle administration group, group 2: 50 mg/kg Example 27 compound administration group). The drug was administered once a day for 12 times. To evaluate therapeutic efficacy, tumor volume was measured before drug administration and on days 4, 7, 10, 13, and 15 after drug administration. Tumor volume (mm³) was calculated as follows (tumor volume (mm³) = (A X B²)/2, where A = tumor long axis, B = tumor short axis). Body weight was measured before administration and on days 4, 7, 10, 13, and 15 after drug administration during the drug administration period. Tumor was excised on day 15 after drug treatment and body weight was measured.

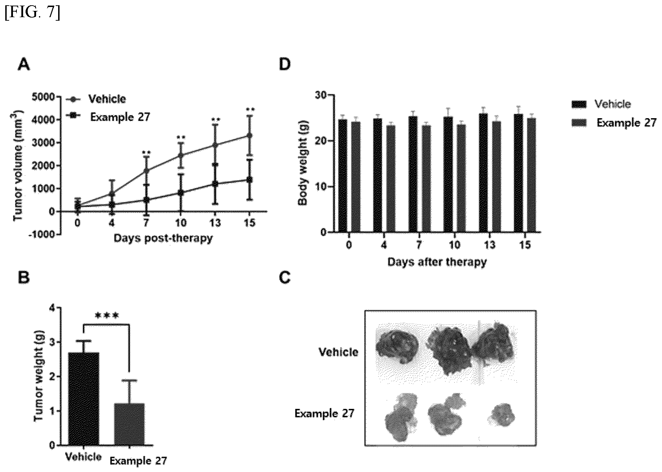

As a result, the vehicle administration group showed significant tumor growth until day 15 after administration, but the 50 mg/kg of Example 27 compound administration group showed a tumor growth inhibitory effect, and its statistical significance was confirmed (**p<0.005, compared with vehicle) (FIG. 7A). The tumor was removed on day 15 after drug administration and its weight was measured, and similar to the results of tumor volume measurement, the tumor weight of the vehicle group was significantly higher than that of the Example 27 compound administration group, and statistical significance was also confirmed (***p<0.0005, compared with vehicle) (FIGS. 7B and 7C). No rapid weight loss was observed during the drug administration period (FIG. 7D).

Although the present disclosure has been described in detail through preferred embodiments and experimental examples in the above, the scope of the present disclosure is not limited to the specific embodiments, and should be construed by the appended claims. In addition, those skilled in the art should understand that many modifications and variations are possible without departing from the scope of the present disclosure.

## Claims

1. A compound represented by the following Chemical Formula 1, a stereoisomer thereof, a hydrate thereof, or a salt thereof: 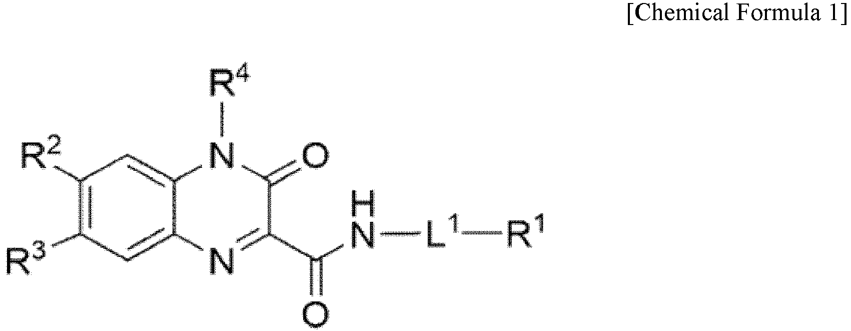
wherein, in the Chemical Formula 1,
R¹ is unsubstituted or substituted Cs-scycloalkyl, unsubstituted or substituted C₆₋₁₀aryl, unsubstituted or substituted heteroaryl of 5 to 8 atoms containing one or more heteroatoms selected from the group consisting of N, O and S, unsubstituted or substituted fused heteroaryl of 8 to 10 atoms containing one or more heteroatoms selected from the group consisting of N, O and S, or C₁₋₁₅alkenyl,
wherein the substituted Cs-scycloalkyl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₁₋₈alkoxycarbonyl, C_{1- 8}alkoxycarbonylC₁₋₅alkyl, and Ci-salkylcarbonyloxy,
the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₆₋₁₀aryl, C₁₋₈alkoxycarbonyl, Ci-salkoxycarbonylCi-salkyl, C₁₋ salkylcarbonyloxy, and heteroaryl of 5 to 8 atoms containing one or more heteroatoms selected from the group consisting of N, O and S,
the substituted heteroaryl of 5 to 8 atoms is substituted with halogen, or C₆₋₁₀aryl substituted with halogen, and
the substituted fused heteroaryl of 8 to 10 atoms is substituted with one or more substituents selected from the group consisting of -OH, halogen, C₁₋₈alkoxycarbonyl, C₁₋₈alkoxycarbonylC₁₋₅alkyl, and Ci-salkylcarbonyloxy;
R² and R³ are each independently -H, halogen, or C₁₋₁₀alkyl;
R⁴ is Ci-salkyl, or C₃₋₁₀alkyl substituted with two or more -OH; and
L¹ is a bond, or C₁₋₁₀alkylene.

2. The compound of claim 1, a stereoisomer thereof, a hydrate thereof, or a salt thereof, wherein the R¹ is unsubstituted or substituted C₅₋₆cycloalkyl, unsubstituted or substituted C₆₋₈aryl, unsubstituted or substituted heteroaryl of 5 to 6 atoms containing one or more heteroatoms selected from the group consisting of N, O and S, unsubstituted or substituted fused heteroaryl of 8 to 10 atoms containing one or more heteroatoms selected from the group consisting of N, O and S, or C₅₋₁₅alkenyl,
wherein the substituted C₅₋₆cycloalkyl is substituted with one or more substituents selected from the group consisting of halogen, -CONH-OH, -CH₂-CONH-OH, -CH₂CH₂-CONH-OH, C₁₋₈alkyl, Ci-salkoxy, C₁₋₈haloalkyl, C₁₋₆alkoxycarbonyl, C₁₋₆alkoxycarbonylC₁₋₃alkyl, and C₁₋₆alkylcarbonyloxy,
the substituted C₆₋₈aryl is substituted with one or more substituents selected from the group consisting of halogen, -CONH-OH, -CH₂-CONH-OH, -CH₂CH₂-CONH-OH, C₁₋₈alkyl, Ci-salkoxy, C₁₋₈haloalkyl, C₆₋₈aryl, C₁₋₆alkoxycarbonyl, C₁₋₆alkoxycarbonylC₁₋₃alkyl, C₁₋₆alkylcarbonyloxy, and heteroaryl of 5 to 6 atoms containing one or more heteroatoms selected from the group consisting of N, O and S,
the substituted heteroaryl of 5 to 6 atoms is substituted with halogen, or C₆₋₈aryl substituted with halogen, and
the substituted fused heteroaryl of 8 to 10 atoms is substituted with one or more substituents selected from the group consisting of -OH, halogen, C₁₋₆alkoxycarbonyl, C₁₋₆alkoxycarbonylC₁₋₃alkyl, and C₁₋₆alkylcarbonyloxy.

3. The compound of claim 1, a stereoisomer thereof, a hydrate thereof, or a salt thereof, wherein the R¹ is unsubstituted or substituted C₅₋₆cycloalkyl, unsubstituted or substituted C₆₋₈aryl, unsubstituted or substituted heteroaryl of 5 to 6 atoms containing one or more heteroatoms selected from the group consisting of N, O and S, unsubstituted or substituted fused heteroaryl of 8 to 10 atoms containing one or more heteroatoms selected from the group consisting of N, O and S, or C₆₋₁₂alkenyl,
wherein the substituted C₅₋₆cycloalkyl is substituted with one or more substituents selected from the group consisting of halogen, -CONH-OH, -CH₂-CONH-OH, -CH₂CH₂-CONH-OH, Ci-salkyl, Ci-shaloalkyl, and C₁₋₈alkoxycarbonyl,
the substituted C₆₋₈aryl is substituted with one or more substituents selected from the group consisting of halogen, -CONH-OH, -CH₂-CONH-OH, -CH₂CH₂-CONH-OH, C₁₋₅alkyl, C₁₋₅alkoxy, Ci-shaloalkyl, C₆aryl, C₁₋₈alkoxycarbonyl, C₁₋₅alkoxycarbonylC₁₋₃alkyl, C₁₋salkylcarbonyloxy, and heteroaryl of 5 to 6 atoms containing one or more heteroatoms selected from the group consisting of N, O and S,
the substituted heteroaryl of 5 to 6 atoms is substituted with halogen, or C₆aryl substituted with halogen, and
the substituted fused heteroaryl of 8 to 10 atoms is substituted with one or more substituents selected from the group consisting of -OH, halogen, C₁₋₈alkoxycarbonyl, C₁₋₅alkoxycarbonylC₁₋₃alkyl, and Ci-salkylcarbonyloxy.

4. The compound of claim 1, a stereoisomer thereof, a hydrate thereof, or a salt thereof, wherein the R¹ is 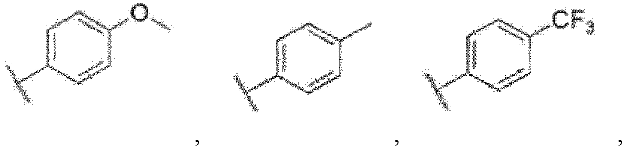 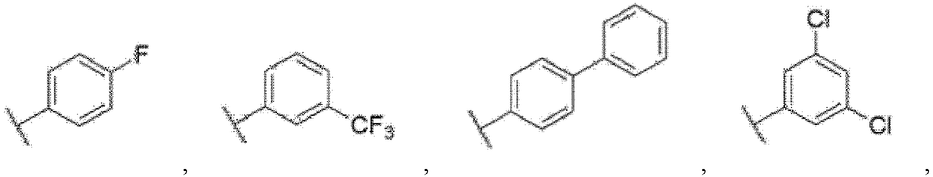 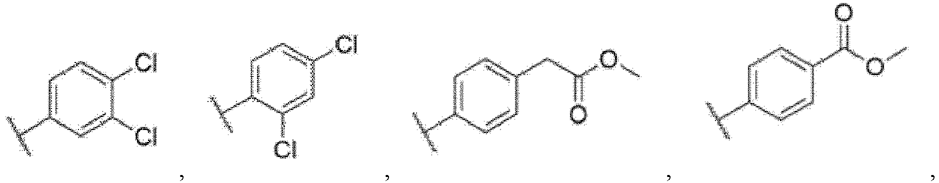 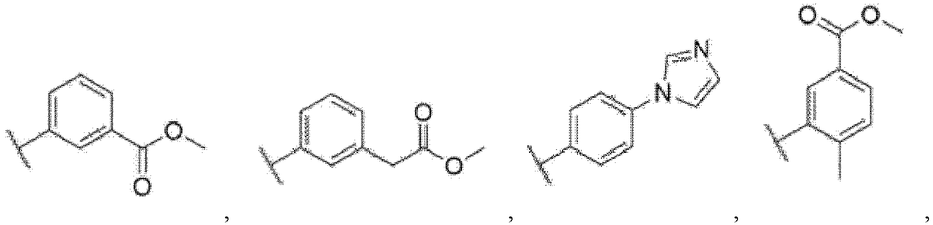 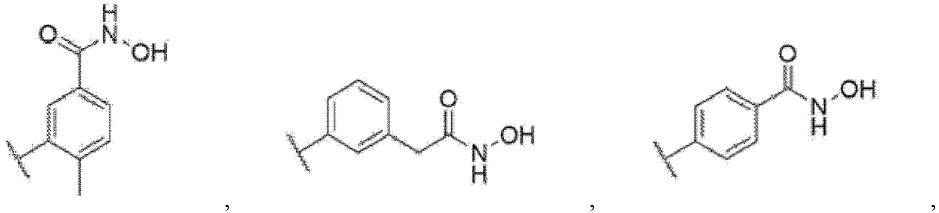 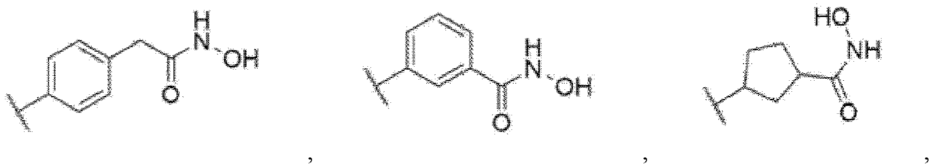 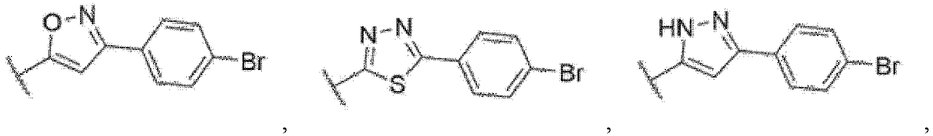 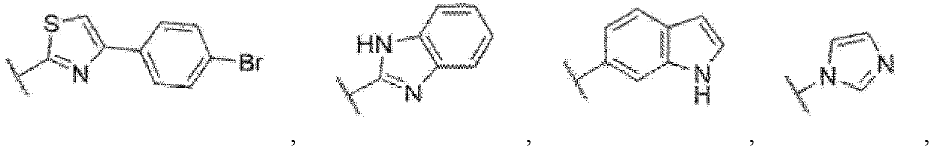 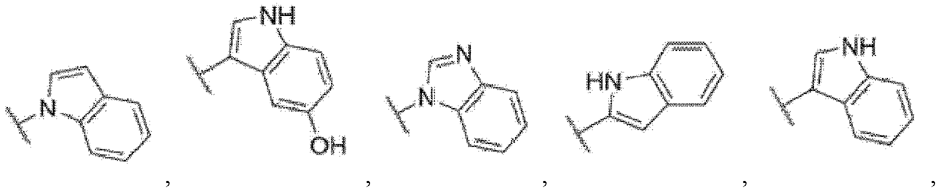 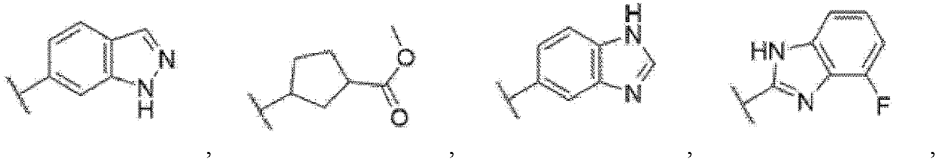 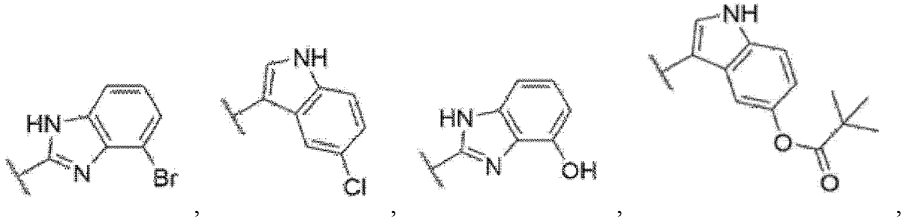 or

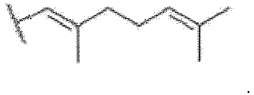

5. The compound of claim 1, a stereoisomer thereof, a hydrate thereof, or a salt thereof, wherein the R² and R³ are each independently -H, halogen, or Ci-salkyl.

6. The compound of claim 1, a stereoisomer thereof, a hydrate thereof, or a salt thereof, wherein the L¹ is a bond, or Ci-salkylene.

7. The compound of claim 1, a stereoisomer thereof, a hydrate thereof, or a salt thereof, wherein the R⁴ is Ci-salkyl, or C₃₋salkyl substituted with 3 to 5 -OH.

8. The compound of claim 1, a stereoisomer thereof, a hydrate thereof, or a salt thereof, wherein the compound represented by the Chemical Formula 1 is a compound represented by the following Chemical Formula 2: 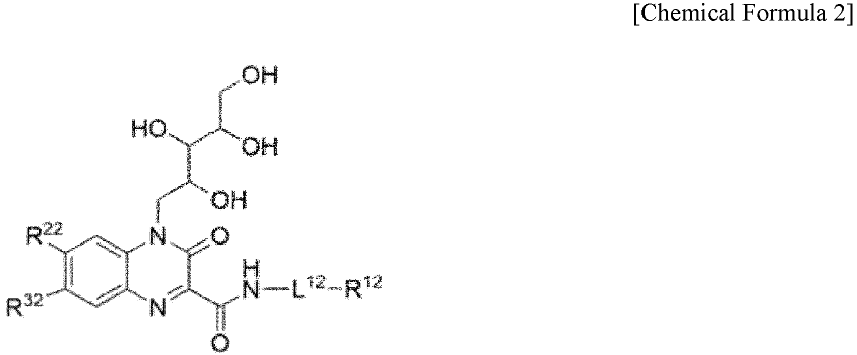
wherein, in the Chemical Formula 2,
R¹² is unsubstituted or substituted C₅₋₈ cycloalkyl, unsubstituted or substituted C₆₋₁₀aryl, unsubstituted or substituted heteroaryl of 5 to 8 atoms containing one or more heteroatoms selected from the group consisting of N, O and S, unsubstituted or substituted fused heteroaryl of 8 to 10 atoms containing one or more heteroatoms selected from the group consisting of N, O and S, or C₁₋₁₅alkenyl,
wherein the substituted Cs-scycloalkyl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₁₋₈alkoxycarbonyl, C₁₋₈alkoxycarbonylC₁₋₅alkyl, and Ci-salkylcarbonyloxy,
the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₆₋₁₀aryl, C₁₋₈alkoxycarbonyl, Ci-salkoxycarbonylCi-salkyl, C₁₋salkylcarbonyloxy, and heteroaryl of 5 to 8 atoms containing one or more heteroatoms selected from the group consisting of N, O and S,
the substituted heteroaryl of 5 to 8 atoms is substituted with halogen, or C₆₋₁₀aryl substituted with halogen, and
the substituted fused heteroaryl of 8 to 10 atoms is substituted with one or more substituents selected from the group consisting of -OH, halogen, C₁₋₈alkoxycarbonyl, C₁₋₈alkoxycarbonylC₁₋₅alkyl, and C₁₋₈alkylcarbonyloxy;
R²² and R³² are each independently -H, halogen, or C₁₋₁₀alkyl; and
L¹² is a bond, or C₁₋₁₀alkylene.

9. The compound of claim 1, a stereoisomer thereof, a hydrate thereof, or a salt thereof, wherein the compound represented by the Chemical Formula 1 is a compound represented by the following Chemical Formula 3: 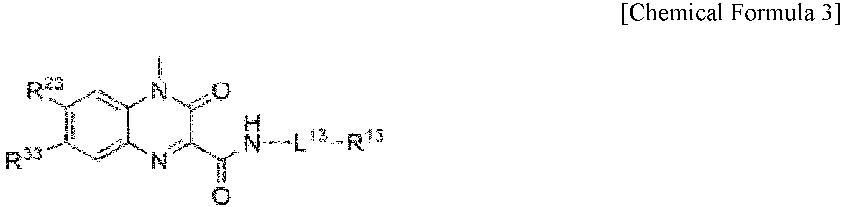
wherein, in the Chemical Formula 3,
R¹³ is unsubstituted or substituted C₆₋₁₀aryl, or unsubstituted or substituted fused heteroaryl of 8 to 10 atoms containing one or more heteroatoms selected from the group consisting of N, O and S,
wherein the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀haloalkyl, Ci-salkoxycarbonyl, Ci-salkoxycarbonylCi-salkyl, and C₁₋salkylcarbonyloxy, and
the substituted fused heteroaryl of 8 to 10 atoms is substituted with one or more substituents selected from the group consisting of -OH and halogen;
R²³ and R³³ are each independently -H, halogen, or C₁₋₁₀alkyl; and
L¹³ is a bond, or C₁₋₁₀alkylene.

10. The compound of claim 1, a stereoisomer thereof, a hydrate thereof, or a salt thereof, wherein the compound represented by the Chemical Formula 1 is any one selected from the following group of compounds:
(1) N-(4-methoxybenzyl)-6,7-dimethyl-3-oxo-4-((2S,3 S,4R)-2,3,4,5-tetrahydroxypentyl)-3,4-dihydroquinoxaline-2-carboxamide;
(2) 6,7-dimethyl-N-(4-methylbenzyl)-3-oxo-4-((2S,3S,4R)-2,3,4,5-tetrahydroxypentyl)-3,4-dihydroquinoxaline-2-carboxamide;
(3) 6,7-dimethyl-3-oxo-4-((2S,3 S,4R)-2,3,4,5-tetrahydroxypentyl)-N-(4-(trifluoromethyl)benzyl)- 3,4-dihydroquinoxaline-2-carboxamide;
(4) N-(4-fluorobenzyl)-6,7-dimethyl-3-oxo-4-((2S,3S,4R)-2,3,4,5-tetrahydroxypentyl)-3 ,4-dihydroquinoxaline-2-carboxamide;
(5) 6,7-dimethyl-3-oxo-4-((2S,3 S,4R)-2,3,4,5-tetrahydroxypentyl)-N-(3-(trifluoromethyl) Benzyl)-3,4-dihydroquinoxaline-2-carboxamide;
(6) N-([1,1'-biphenyl]-4-ylmethyl)-6,7-dimethyl-3-oxo-4-((2S,3S,4R)-2,3,4,5-tetrahydroxypentyl)-3,4-dihydroquinoxaline-2-carboxamide;
(7) N-(3,5-dichlorobenzyl)-6,7-dimethyl-3-oxo-4-((2S,3 S,4R)-2,3,4,5-tetrahydroxypentyl)-3,4-dihydroquinoxaline-2-carboxamide;
(8) N-(3,4-dichlorobenzyl)-6,7-dimethyl-3-oxo-4-((2S,3 S,4R)-2,3,4,5-tetrahydroxypentyl)-3,4-dihydroquinoxaline-2-carboxamide;
(9) N-(2,4-dichlorophenethyl)-6,7-dimethyl-3-oxo-4-((2S,3 S,4R)-2,3,4,5-tetrahydroxypentyl )-3,4-dihydroquinoxaline-2-carboxamide;
(10) N-(3-(4-bromophenyl)isoxazol-5-yl)-6,7-dimethyl-3-oxo-4-((2S,3S,4R)-2,3,4,5-tetrahydroxypentyl)-3,4-dihydroquinoxaline-2-carboxamide;
(11) N-(5-(4-bromophenyl)-1,3,4-thiadiazol-2-yl)-6,7-dimethyl-3-oxo-4-((2S,3S ,4R)-2,3,4,5-tetrahydroxypentyl)-3,4-dihydroquinoxaline-2-carboxamide;
(12) N-(3-(4-bromophenyl)-1H-pyrazol-5-yl)-6,7-dimethyl-3-oxo-4-((2S,3S,4R)-2,3,4,5-tetrahydroxypentyl)-3,4-dihydroquinoxaline-2-carboxamide;
(13) N-(4-(4-bromophenyl)thiazol-2-yl)-6,7-dimethyl-3-oxo-4-((2S,3S,4R)-2,3,4,5-tetrahydroxypentyl)-3,4-dihydroquinoxaline-2-carboxamide;
(14) N-((E)-3,7-dimethylocta-2,6-dien-1-yl)-6,7-dimethyl-3-oxo-4-((2S,3S,4R)-2,3,4,5-tetrahydroxypentyl)-3,4-dihydroquinoxaline-2-carboxamide;
(15) Methyl 2-(4-((6,7-dimethyl-3-oxo-4-((2S,3S,4R)-2,3,4,5-tetrahydroxypentyl)-3,4 -dihydroquinoxaline-2-carboxamido)methyl)phenyl)acetate;
(16) Methyl 3-((6,7-dimethyl-3-oxo-4-((2S,3S,4R)-2,3,4,5-tetrahydroxypentyl)-3,4-dihydroquinoxaline-2-carboxamido)methyl)benzoate;
(17) N-(2-(1H-benzo[d]imidazol-2-yl)ethyl)-6,7-dimethyl-3-oxo-4-((2S,3S,4R)-2,3,4,5-tetrahydroxypentyl)-3,4-dihydroquinoxaline-2-carboxamide;
(18) Methyl 4-((6,7-dimethyl-3-oxo-4-((2S,3S,4R)-2,3,4,5-tetrahydroxypentyl)-3,4-dihydroquinoxaline-2-carboxamido)methyl)benzoate;
(19) Methyl 2-(3-(6,7-dimethyl-3-oxo-4-((2S,3S,4R)-2,3,4,5-tetrahydroxypentyl)-3,4-dihydroquinoxaline-2-carboxamido)phenyl)acetate;
(20) Methyl 4-(6,7-dimethyl-3-oxo-4-((2S,3S,4R)-2,3,4,5-tetrahydroxypentyl)-3,4-dihydroquinoxaline-2-carboxamido)benzoate;
(21) N-(3-(1H-benzo[d]imidazol-2-yl)propyl)-6,7-dimethyl-3-oxo-4-((2S,3S,4R)-2,3,4,5-tetrahydroxypentyl)-3,4-dihydroquinoxaline-2-carboxamide;
(22) N-((1H-indol-6-yl)methyl)-6,7-dimethyl-3-oxo-4-((2S,3S,4R)-2,3,4,5-tetrahydroxypentyl)-3,4-dihydroquinoxaline-2-carboxamide;
(23) N-(4-(1H-imidazol-1-yl)phenyl)-6,7-dimethyl-3-oxo-4-((2S,3S,4R)-2,3,4,5-tetrahydroxypentyl)-3,4-dihydroquinoxaline-2-carboxamide;
(24) N-((1H-benzo[d]imidazol-2-yl)methyl)-6,7-dimethyl-3-oxo-4-((2S,3S,4R)-2,3,4,5-tetrahydroxypentyl)-3,4-dihydroquinoxaline-2-carboxamide;
(25) N-(3-1H-imidazol-1-yl)propyl)-6,7-dimethyl-3-oxo-4-((2S,3S,4R)-2,3,4,5-tetrahydroxypentyl)-3,4-dihydroquinoxaline-2-carboxamide;
(26) N-(3-(1H-indol-1-yl)propyl)-6,7-dimethyl-3-oxo-4-((2S,3S,4R)-2,3,4,5-tetrahydroxypentyl)-3,4-dihydroquinoxaline-2-carboxamide;
(27) N-(2-(5-hydroxy-1H-indol-3-yl)ethyl)-6,7-dimethyl-3-oxo-4-((2S,3S,4R)-2,3,4,5-tetrahydroxypentyl)-3,4-dihydroquinoxaline-2-carboxamide;
(28) N-(3-(1H-benzo[d]imidazol-1-yl)propyl)-6,7-dimethyl-3-oxo-4-((2S,3S,4R)-2,3,4,5-tetrahydroxypentyl)-3,4,-dihydroquinoxaline-2-carboxamide;
(29) N-(2-(1H-indol-2-yl)ethyl)-6,7-dimethyl-3-oxo-4-((2S,3S,4R)-2,3,4,5-tetrahydroxypentyl)-3,4-dihydroquinoxaline-2-carboxamide;
(30) Methyl 3-(6,7-dimethyl-3-oxo-4-((2S,3S,4R)-2,3,4,5-tetrahydroxypentyl)-3,4-dihydroquinoxaline-2-carboxamido)-4-methylbenzoate;
(31) N-(3-(1H-indol-3-yl)propyl)-6,7-dimethyl-3-oxo-4-((2S,3S,4R)-2,3,4,5-tetrahydroxypentyl)-3,4-dihydroquinoxaline-2-carboxamide;
(32) N-(1H-indol-5-yl)-6,7-dimethyl-3-oxo-4-((2S,3S,4R)-2,3,4,5-tetrahydroxypentyl)-3,4-dihydroquinoxaline-2-carboxamide;
(33) Methyl 3-(6,7-dimethyl-3-oxo-4-((2S,3S,4R)-2,3,4,5-tetrahydroxypentyl)-3,4-dihydroquinoxaline-2-carboxamido)cyclopentane-1 -carboxylate;
(34) N-((1H-benzo[d]imidazol-5-yl)methyl)-6,7-dimethyl-3-oxo-4-((2S,3S,4R)-2,3,4,5-tetrahydroxypentyl)-3,4-dihydroquinoxaline-2-carboxamide;
(35) N-(4-fluoro-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide;
(36) N-(4-bromo-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide;
(37) N-(2-(5-chloro-1H-indol-3-yl)ethyl)-6,7-dimethyl-3-oxo-4-((2S,3S,4R)-2,3,4,5-tetrahydroxypentyl)-3,4-dihydroquinoxaline-2-carboxamide;
(38) N-(2-(4-fluoro-1H-benzo[d]imidazol-2-yl)ethyl)-6,7-dimethyl-3-oxo-4-((2S,3S,4R)-2,3,4,5-tetrahydroxypentyl)-3,4-dihydroquinoxaline-2-carboxamide;
(39) N-(4-hydroxy-1H-benzo[d]imidazol-2-yl)-6,7-dimethyl-3-oxo-4-((2S,3S,4R)-2,3,4,5-tetrahydroxypentyl)-3,4-dihydroquinoxaline-2-carboxamide;
(40) 3-(2-(6,7-dimethyl-3-oxo-4-((2S,3S,4R)-2,3,4,5-tetrahydroxypentyl)-3,4-dihydroquinoxaline-2-carboxamido)ethyl)-1H-indol-5-yl pivalate;
(41) N-(2-(5-hydroxy-1H-indol-3-yl)ethyl)-4,6,7-trimethyl-3-oxo-3,4-dihydroquinoxaline-2-carboxamide;
(42) N-(2-(4-fluoro-1H-benzo[d]imidazol-2-yl)ethyl)-4,6,7-trimethyl-3-oxo-3,4-dihydroquinoxaline-2-carboxamide;
(43) N-(2-(5-chloro-1H-indol-3-yl)ethyl)-4,6,7-trimethyl-3-oxo-3,4-dihydroquinoxaline-2-carboxamide;
(44) methyl 4-methyl-3-(4,6,7-trimethyl-3-oxo-3,4-dihydroquinoxaline-2-carboxamido)benzoate;
(45) N-(5-(hydroxycarbamoyl)-2-methylphenyl)-4,6,7-trimethyl-3-oxo-3,4-dihydroquinoxaline-2-carboxamide;
(46) N-(3-(2-(hydroxyamino)-2-oxoethyl)phenyl)-6,7-dimethyl-3-oxo-4-((2S,3S,4R)-2,3,4,5-tetrahydroxypentyl)-3,4-dihydroquinoxaline-2-carboxamide;
(47) N-(4-(hydroxycarbamoyl)benzyl)-6,7-dimethyl-3-oxo-4-((2S,3S,4R)-2,3,4,5-tetrahydroxypentyl)-3,4-dihydroquinoxaline-2-carboxamide;
(48) N-(4-(hydroxycarbamoyl)phenyl)-6,7-dimethyl-3-oxo-4-((2S,3S,4R)-2,3,4,5-tetrahydroxypentyl)-3,4-dihydroquinoxaline-2-carboxamide;
(49) N-(4-(2-(hydroxyamino)-2-oxoethyl)benzyl)-6,7-dimethyl-3-oxo-4-((2S,3S,4R)-2,3,4,5-tetrahydroxypentyl)-3,4-dihydroquinoxaline-2-carboxamide;
(50) N-(3-(hydroxycarbamoyl)benzyl)-6,7-dimethyl-3-oxo-4-((2S,3S,4R)-2,3,4,5-tetrahydroxypentyl)-3,4-dihydroquinoxaline-2-carboxamide;
(51) N-(5-(hydroxycarbamoyl)-2-methylphenyl)-6,7-dimethyl-3-oxo-4-((2S,3S,4R)-2,3,4,5- tetrahydroxypentyl)-3,4-dihydroquinoxaline-2-carboxamide; and
(52) N-(3-(hydroxycarbamoyl)cyclopentyl)-6,7-dimethyl-3-oxo-4-((2S,3S,4R)-2,3,4,5-tetrahydroxypentyl)-3,4-dihydroquinoxaline-2-carboxamide.

11. A method of preparing a compound represented by Chemical Formula 1, comprising:
as shown in the following Reaction Scheme 1,
reacting a compound represented by Chemical Formula 1A with a hydroxide ion (OH⁻ ) to prepare a compound represented by Chemical Formula 1B; and
reacting the compound represented by Chemical Formula 1B with a compound represented by Chemical Formula 1C to prepare a compound represented by Chemical Formula 1, 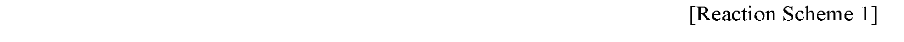 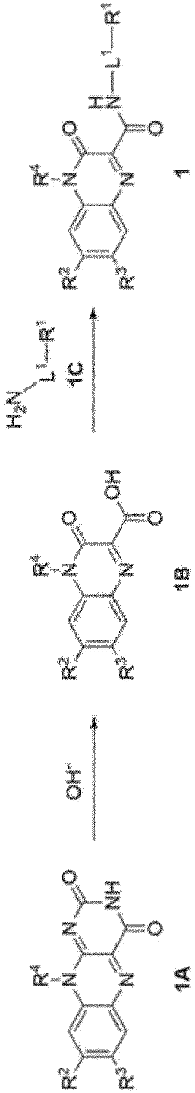
wherein, in the Reaction Scheme 1,
R¹ is unsubstituted or substituted Cs-scycloalkyl, unsubstituted or substituted C₆₋₁₀aryl, unsubstituted or substituted heteroaryl of 5 to 8 atoms containing one or more heteroatoms selected from the group consisting of N, O and S, unsubstituted or substituted fused heteroaryl of 8 to 10 atoms containing one or more heteroatoms selected from the group consisting of N, O and S, or C₁₋₁₅alkenyl,
wherein the substituted Cs-scycloalkyl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₁₋₈alkoxycarbonyl, C₁₋₈alkoxycarbonylC₁₋₅alkyl, and C₁₋₈alkylcarbonyloxy,
the substituted C₆₋₁₀aryl is substituted with one or more substituents selected from the group consisting of halogen, -CONH-OH, -CH₂-CONH-OH, -CH₂CH₂-CONH-OH, C₁₋₁₀alkyl, C₁₋₁₀alkoxy, C₁₋₁₀haloalkyl, C₆₋₁₀aryl, C₁₋₈alkoxycarbonyl, Ci-salkoxycarbonylCi-salkyl, C₁₋salkylcarbonyloxy, and heteroaryl of 5 to 8 atoms containing one or more heteroatoms selected from the group consisting of N, O and S,
the substituted heteroaryl of 5 to 8 atoms is substituted with halogen, or C₆₋₁₀aryl substituted with halogen, and
the substituted fused heteroaryl of 8 to 10 atoms is substituted with one or more substituents selected from the group consisting of -OH, halogen, C₁₋₈alkoxycarbonyl, C₁₋₈alkoxycarbonylC₁₋₅alkyl, and C₁₋₈alkylcarbonyloxy;
R² and R³ are each independently -H, halogen, or C₁₋₁₀alkyl;
R⁴ is C₁₋₈alkyl, or C₃₋₁₀alkyl substituted with two or more -OH; and
L¹ is a bond, or C₁₋₁₀alkylene.

12. A pharmaceutical composition for preventing or treating cancer disease, comprising the compound according to claim 1, a stereoisomer thereof, a hydrate thereof, or a pharmaceutically acceptable salt thereof as an active ingredient.

13. The pharmaceutical composition of claim 12, wherein the cancer disease is selected from the group consisting of prostate cancer, liver cancer, lung cancer, breast cancer, ovarian cancer, uterine cancer, pancreatic cancer, lung cancer, stomach cancer, colon cancer, skin cancer, head or neck cancer, brain cancer, larynx cancer, bladder cancer, esophagus cancer, thyroid cancer, kidney cancer, and rectal cancer.

14. The pharmaceutical composition of claim 12, wherein the pharmaceutical composition selectively inhibits histone deacetylase 8 (HDAC8).

15. A health functional food composition for preventing or ameliorating cancer disease, comprising the compound according to claim 1, a stereoisomer thereof, a hydrate thereof, or a pharmaceutically acceptable salt thereof as an active ingredient.
